# EUROPEAN PATENT SPECIFICATION

(11) **EP 0 626 957 B1**
(45) Date of publication and mention of the grant of the patent: **03.03.1999**
(21) Application number: 94902933.4
(22) Date of filing: 22.12.1993
(51) Int. Cl.: C07D 405/06, C07D 409/06, C07D 233/64, C07D 213/30, C07D 213/38, C07D 213/55, C07D 213/36, C07D 213/40

(54) **STYRIL DERIVATIVES AND PROCESSES FOR THEIR PREPARATION**
STYRYLDERIVATE UND VERFAHREN ZU IHRER HERSTELLUNG
DERIVES DE STYRYL ET PROCEDES POUR LEUR PREPARATION

(30) Priority: 23.12.1992 GB 9226830
(43) Date of publication of application: 07.12.1994
(73) Proprietor: CELLTECH THERAPEUTICS LIMITED, Slough, Berkshire SL1 4EN (GB)
(72) Inventor: WARRELLOW, Graham John, Northwood, Middlesex HA6 3QU (GB); BOYD, Ewan Campbell, Slough, Berkshire SL1 9HB (GB); ALEXANDER, Rikki Peter, Bucks HP12 3HY (GB)
(74) Representative: Skailes, Humphrey John
(86) International application number: PCT/GB93/02627
(87) International publication number: WO 94/14800

(56) References cited:
- EP-A- 0 490 823
- WO-A-91/15451
- WO-A-92/00968
- DE-A- 2 501 443
- JOURNAL OF ORGANIC CHEMISTRY vol. 23, 1958, pages 1261 - 1263 N.P. BUU-HOI ET AL 'Bromination of some 1,2,3-triarylethylenes'
- JOURNAL OF HETEROCYCLIC CHEMISTRY vol. 16, no. 4, 1979, pages 711 - 715 M.S. MANHAS ET AL 'Heterocyclic compounds XII. Quinazoline derivatives as potential antifertility agents'
- CHEMICAL ABSTRACTS, vol. 118, 1993, Columbus, Ohio, US; abstract no. 136183z, HIROSE ET AL 'Styrene derivatives and electrophotographic photoreceptor containing them' page 793 ;
- CHEMICAL ABSTRACTS, vol. 93, 1980, Columbus, Ohio, US; abstract no. 95160j, MEZHERITSKAYA L. V ET AL 'Synthesis and properties of carboxonium heterocyclic systems' page 635 ;
- JOURNAL OF MEDICINAL CHEMISTRY vol. 29, no. 8, 1986, pages 1355 - 1362 M.R.SCHNEIDER ET AL 'Catechol estrogens of the 1,1,2-triphenylbut-1-ene type'

## Description

This invention relates to a novel series of styryl derivatives, to processes for their preparation, to pharmaceutical compositions containing them, and to their use in medicine.

Many hormones and neurotransmitters modulate tissue function by elevating intra-cellular levels of adenosine 3', 5'-cyclic monophosphate (cAMP). The cellular levels of cAMP are regulated by mechanisms which control synthesis and breakdown. The synthesis of cAMP is controlled by adenyl cyclase which may be directly activated by agents such as forskolin or indirectly activated by the binding of specific agonists to cell surface receptors which are coupled to adenyl cyclase. The breakdown of cAMP is controlled by a family of phosphodiesterase (PDE) isoenzymes, which also control the breakdown of guanosine 3',5'-cyclic monophosphate (cGMP). To date, seven members of the family have been described (PDE I-VII) the distribution of which varies from tissue to tissue. This suggests that specific inhibitors of PDE isoenzymes could achieve differential elevation of cAMP in different tissues, [for reviews of PDE distribution, structure, function and regulation, see Beavo & Reifsnyder (1990) TIPS, 11: 150-155 and Nicholson et al (1991) TIPS, 12: 19-27].

There is clear evidence that elevation of cAMP in inflammatory leukocytes leads to inhibition of their activation. Furthermore, elevation of cAMP in airway smooth muscle has a spasmolytic effect. In these tissues, PDE IV plays a major role in the hydrolysis of cAMP. It can be expected, therefore, that selective inhibitors of PDE IV would have therapeutic effects in inflammatory diseases such as asthma, by achieving both anti-inflammatory and bronchodilator effects.

The design of PDE IV inhibitors has met with limited success to date, in that many of the potential PDE IV inhibitors which have been synthesised have lacked potency and/or have been capable of inhibiting more than one type of PDE isoenzyme in a non-selective manner. Lack of a selective action has been a particular problem given the widespread role of cAMP in vivo and what is needed are potent selective PDE IV inhibitors with an inhibitory action against PDE IV and little or no action against other PDE isoenzymes.

We have now found a novel series of styryl derivatives, members of which compared to known structurally similar compounds are potent inhibitors of PDE IV at concentrations at which they have little or no inhibitory action on other PDE isoenzymes. These compounds inhibit the isolated PDE IV enzyme and also elevate cAMP in isolated leukocytes. Certain compounds prevent inflammation in the lungs induced by carrageenan, platelet-activating factor (PAF), interleukin-5 (IL-5) or antigen challenge. These compounds also suppress the hyper-responsiveness of airway smooth muscle seen in inflamed lungs. Advantageously, compounds according to the invention have good oral activity and at orally effective doses exhibit little or none of the side-effects associated with known PDE IV inhibitors, such as rolipram. The compounds of the invention are therefore of use in medicine, especially in the prophylaxis and treatment of asthma.

Thus according to one aspect of the invention, we provide a compound of formula (1) 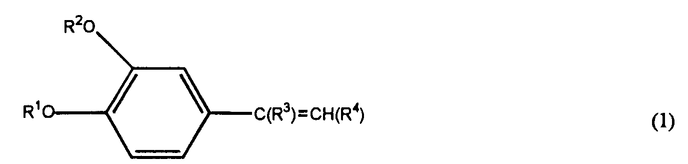
wherein R¹ is a methyl group optionally substituted by one, two or three fluorine or chlorine atoms; R² is a C₃₋₈ cycloalkyl group optionally substituted by one, two or three substituents selected from halogen atoms, C₁₋₆ alkyl, hydroxyl or C₁₋₆ alkoxy group;
R³ and R⁴, which may be the same or different, is each a group Ar, where Ar is a phenyl, pyridyl, pyridazinyl, pyrimidinyl or pyrazinyl group, each of said groups being optionally substituted by one, two or more substituents selected from an atom or group R⁸ or Alk¹H or -Alk¹(R⁸)ₘ wherein R⁸ is a halogen atom, or an amino (-NH₂), substituted amino, nitro, cyano, hydroxyl (-OH), substituted hydroxyl, cycloalkoxy, formyl [HC(O)-], carboxyl (-CO₂H), esterified carboxyl, thiol (-SH), substituted thiol, -C(O)Alk¹H, -SO₃H, -SO₂Alk¹H, -SO₂NH₂, -SO₂NHAlk¹H, -SO₂N[Alk¹H]₂, -CONH₂, -CONHAlk¹H, -CON[Alk¹H]₂, -NHSO₂H, -NHSO₂Alk¹H, -N[SO₂Alk¹H]₂, -NHSO₂NH₂, -NHSO₂NHAlk¹H, -NHSO₂N[Alk¹H]₂, -NHC(O)Alk¹H, or -NHC(O)OAlk¹H group; Alk¹ is a straight or branched C₁₋₆alkylene, C₂₋₆alkenylene, or C₂₋₆alkynylene chain optionally interrupted by one, two or three -O- or -S- atoms or -S(O)p- [where p is an integer 1 or 2] or N(R⁶)- [where R⁶ is a hydrogen atom or C₁₋₆alkyl group] groups;
and m is zero or an integer 1, 2 or 3;
said substituted amino group being a group -NH[Alk¹(R^{8a})ₘ] [where Alk¹ and m are as defined above and R^{8a} is as defined above for R⁸ but is not a substituted amino, a substituted hydroxyl or a substituted thiol group] or a group -N[Alk¹(R^{8a})ₘ]₂ wherein each -Alk¹(R^{8a})ₘ group is the same or different;
said substituted hydroxyl or substituted thiol group being a group -OAlk¹(R^{8a})ₘ or -SAlk¹(R^{8a})ₘ respectively, where Alk¹, R^{8a} and m are as just defined; and the salts, solvates, hydrates and N-oxides thereof.

The compounds of formula (1) exist as geometrical isomers and the invention extends to all such individual isomers and mixtures thereof. Formula (1) and the formulae hereinafter should be understood to include all individual isomers and mixtures thereof, unless stated otherwise, and even though only one isomer may be depicted.

Particular substituted R¹ groups include for example -CH₂F, -CH₂Cl, -CHF₂, -CHCl₂, -CF₃ or -CCl₃ groups.

R² in the compounds of formula (1) may be for example a cyclobutyl, cyclopentyl or cycloheryl group optionally substituted by one, two or three substituents selected from halogen atoms, e.g. fluorine, chlorine, bromine or iodine atoms, straight or branched C₁₋₆alkyl e.g. C₁₋₃alkyl such as methyl or ethyl, hydroxyl or C₁₋₆alkoxy e.g. C₁₋₃alkoxy such as methoxy or ethoxy groups.

The heteroaryl group represented by Ar may be attached to the remainder of the molecule of formula (1) through any ring carbon or heteroatom as appropriate. Thus, for example, when the group Ar is a pyridyl group it may be a 2-pyridyl, 3-pyridyl or 4-pyridyl group.

When in compounds of formula (1) the Ar group is a nitrogen-containing heterocycle it may be possible to form quaternary salts, for example N-alkyl quaternary salts and the invention is to be understood to extend to such salts. Thus for example when the group Ar is a pyridyl group, pyridinium salts may be formed, for example N-alkylpyridinium salts such as N-methylpyridinium.

When in the group -Alk¹(R⁸)ₘ m is an integer 1, 2 or 3, it is to be understood that the substituent or substituents R⁸ may be present on any suitable carbon atom in -Alk¹. Where more than one R⁸ substitutent is present these may be the same or different and may be present on the same or different carbon atom in Alk¹. Clearly, when m is zero and no substituent R⁸ is present or when Alk¹ forms part of a group such as -SO₂Alk¹ the alkylene, alkenylene or alkynylene chain represented by Alk¹ becomes an alkyl, alkenyl or alkynyl group.

When R⁸ is a substituted amino group it may be a group -NH[Alk¹ (R^{8a})ₘ] [where Alk¹ and m are as defined above and R^{8a} is as defined above for R⁸ but is not a substituted amino, a substituted hydroxyl or a substituted thiol group] or a group -N[Alk¹(R^{8a})ₘ]₂ wherein each -Alk¹(R^{8a})ₘ group is the same or different.

When R⁸ is a halogen atom it may be for example a fluorine, chlorine, bromine, or iodine atom.

When R⁸ is a cycloalkoxy group it may be for example a C₅₋₇cycloalkoxy group such as a cyclopentyloxy or cyclohexyloxy group.

When R⁸ is a substituted hydroxyl or substituted thiol group it may be a group -OAlk¹(R^{8a})ₘ or -SAlk¹(R^{8a})ₘ respectively, where Alk¹, R^{8a} and m are as just defined.

Esterified carboxyl groups represented by the group R⁸ include groups of formula -CO₂Alk² wherein Alk² is a straight or branched, optionally substituted C₁₋₈alkyl group such as a methyl, ethyl, n-propyl, i-propyl, n-butyl, i-butyl, s-butyl or t-butyl group; a C₆₋₁₂arylC₁₋₈alkyl group such as an optionally substituted benzyl, phenylethyl, phenylpropyl, 1-naphthylmethyl or 2-naphthylmethyl group; a C₆₋₁₂aryl group such as an optionally substituted phenyl, 1-naphthyl or 2-naphthyl group; a C₆₋₁₂aryloxyC₁₋₈alkyl group such as an optionally substituted phenyloxymethyl, phenyloxyethyl, 1-naphthyloxymethyl, or 2-naphthyloxymethyl group; an optionally substituted C₁₋₈alkanoyloxyC₁₋₈alkyl group, such as a pivaloyloxymethyl, propionyloxyethyl or propionyloxypropyl group; or a C₆₋₁₂aroyloxyC₁₋₈alkyl group such as an optionally substituted benzoyloxyethyl or benzoyloxypropyl group. Optional substituents present on the Alk² group include R⁷ substituents described above.

When Alk¹ is present it may be for example a methylene, ethylene, n-propylene, i-propylene, n-butylene, i-butylene, s-butylene, t-butylene, ethenylene, 2-propenylene, 2-butenylene, 3-butenylene, ethynylene, 2-propynylene, 2-butynylene or 3-butynylene chain, optionally interrupted by one, two, or three -O- or -S-, atoms or -S(O)-, -S(O)₂- or -N(R⁶)- groups.

Particularly useful R³ or R⁴ substituents include fluorine, chlorine, bromine or iodine atoms, or C₁₋₆alkyl, e.g. methyl or ethyl, C₁₋₆alkylamino, e.g. methylamino or ethylamino, C₁₋₆ hydroxyalkyl, e.g. hydroxymethyl or hydroxyethyl, C₁₋₆alkylthiol e.g. methylthiol or ethylthiol, C₁₋₆alkoxy, e.g. methoxy or ethoxy, C₅₋₇cycloalkoxy, e.g. cyclopentyloxy, haloC₁₋₆alkyl, e.g. trifluoromethyl, C₁₋₆alkylamino, e.g. methylamino or ethylamino, amino (-NH₂), aminoC₁₋₆alkyl, e.g. aminomethyl or aminoethyl, C₁₋₆dialkylamino, e.g. dimethylamino or diethylamino, nitro, cyano, hydroxyl (-OH), formyl [HC(O)-], carboxyl (-CO₂H), -CO₂Alk² [where Alk² is as defined above], C₁₋₆ alkanoyl e.g. acetyl, thiol (-SH), thioC₁₋₆alkyl, e.g. thiomethyl or thioethyl, sulphonyl (-SO₃H), C₁₋₆alkylsulphonyl, e.g. methylsulphonyl, aminosulphonyl (-SO₂NH₂), C₁₋₆alkylaminosulphonyl, e.g. methylaminosulphonyl or ethylaminosulphonyl, C₁₋₆dialkylaminosulphonyl, e.g. di-methylaminosulphonyl or diethylaminosulphonyl, carboxamido (-CONH₂), C₁₋₆alkylaminocarbonyl, e.g. methylaminocarbonyl or ethylaminocarbonyl, C₁₋₆dialkylaminocarbonyl, e.g. dimethylaminocarbonyl or diethylaminocarbonyl, sulphonylamino (-NHSO₂H), C₁₋₆alkylsulphonylamino, e.g. methylsulphonylamino or ethylsulphonylamino, C₁₋₆dialkylsulphonylamino, e.g. dimethylsulphonylamino or diethylsulphonylamino, aminosulphonylamino (-NHSO₂NH₂), C₁₋₆alkylaminosulphonylamino, e.g. methylaminosulphonylamino or ethylaminosulphonylamino, C₁₋₆dialkylaminosulphonylamino, e.g. dimethylaminosulphonylamino or diethylaminosulphonylamino, C₁₋₆alkanoylamino, e.g. acetylamino, C₁₋₆alkanoylamino C₁₋₆alkyl, e.g. acetylaminomethyl or C₁₋₆alkoxycarbonylamino, e.g. methoxycarbonylamino, ethoxycarbonylamino or t-butoxycarbonylamino groups.

Where desired, two R³ or R⁴ substituents may be linked together to form a cyclic group such as a cyclic ether, e.g. a C₂₋₆alkylenedioxy group such as ethylenedioxy.

It will be appreciated that where two or more substituents on R³ or R⁴ are present, these need not necessarily be the same atoms and/or groups. The substituents may be present at any ring carbon atom away from that attached to the rest of the molecule of formula (1). Thus, for example, in phenyl groups represented by Ar any substituent may be present at the 2-, 3-, 4-, 5- or 6- positions relative to the ring carbon atom attached to the remainder of the molecule.

In the compounds of formula (1), when an ester group is present, for example a group -CO₂Alk² this may advantageously be a metabolically labile ester.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isethionates, arylsulphonates, e.g. p-toluenesulphonates, besylates or napsylates, phosphates, sulphates, hydrogen sulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

A particularly useful group of compounds of formula (1) has the formula (2): 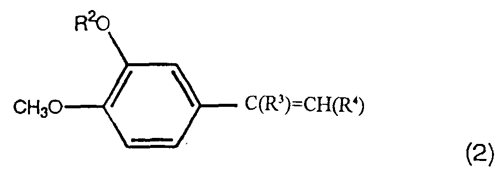 where R², R³ and R⁴ are as defined for formula (1): and the salts, solvates, hydrates and N-oxides thereof.

In the compounds of formulae (1) or (2) R² is preferably an optionally substituted cyclopentyl group. In particular, R² is a cyclopentyl group.

Particularly useful R³ or R⁴ groups include optionally substituted 2-pyridyl, 3-pyridyl or, especially, 4-pyridyl, 3-pyridazinyl, 4-pyridazinyl, 5-pyridazinyl, 2-pyrimidinyl, 4-pyrimidinyl, 5-pyrimidinyl, 2-pyrazinyl, 3-pyrazinyl and phenyl groups.

One particularly useful group of compounds of formulae (1) or (2) is that wherein R³ and R⁴ is each a pyridyl or, especially, a monosubstituted pyridyl, or preferably a distributed pyridyl group, or R³ is a phenyl or substituted phenyl group and R⁴ is a pyridyl or, especially a monosubstituted pyridyl, or preferably a disubstituted pyridyl group.

In this particular group of compounds and also in general in compounds of formulae (1) or (2), when R³ and/or R⁴ is a substituted phenyl group it may be for example a mono-, di- or trisubstituted phenyl group in which the substituent is an atom or group as defined above. When the R³ and/or R⁴ group is a monosubstituted phenyl group the substituent may be in the 2-, or preferably 3-, or especially 4-position relative to the ring carbon atom attached to the remainder of the molecule.

When in compounds of formulae (1) or (2) R³ and/or R⁴ is a substituted pyridyl group it may be for example a mono- or disubstituted pyridyl group, such as a mono- or disubstituted 2-pyridyl, 3-pyridyl or especially 4-pyridyl group substituted by one or two atoms or groups as defined above, in particular one or two halogen atoms such as fluorine or chlorine atoms, or methyl, methoxy, hydroxyl or nitro groups. Particularly useful pyridyl groups of these types are 3-monosubstituted-4-pyridyl or 3,5-disubstituted-4-pyridyl, or 2- or 4-monosubstituted-3-pyridyl or 2,4-disubstituted-3-pyridyl groups.

Compounds in which R³ is an optionally substituted phenyl, or pyridyl in particular 4-pyridyl, group and R⁴ is a pyridyl especially a 4-pyridyl group are particularly preferred.

Particularly useful compounds according to the invention are the (E) and (Z) isomers of
4-[2-(3-Cyclopentyloxy-4-methoxyphenyl)-2-phenylethenyl] pyridine;
4-[1-(3-Cyclopentyloxy-4-methoxyphenyl)-2-(4-pyridyl)ethenyl] pyridine;
4-[2-(3-Cyclopentyloxy-4-methoxyphenyl)-2-(4-fluorophenylethenyl] pyridine;
4-[2-(3-Cyclopentyloxy-4-methoxyphenyl)-2-(4-trifluoromethylphenyl)ethenyl]pyridine;
4-[2-(3-Cyclopentyloxy-4-methoxyphenyl)-2-(2-methoxyphenylethenyl)]pyridine;
4-[2-(3-Cyclopentyloxy-4-methoxyphenyl)-2-(4-methoxyphenyl)ethenyl]pyridine;
4-[2-(3-Cyclopentyloxy-4-methoxyphenyl)-2-(4-methylphenyl) ethenyl]pyridine;
4-[2-(3-Cyclopentyloxy-4-methoxyphenyl)-2-(3-methylphenyl)-ethenyl]pyridine;
4-[2-(3-Cyclopentyloxy-4-methoxyphenyl)-2-(3-cyclopentyloxy-4-methoxyphenyl)ethenyl]pyridine;
4-[2-(3-Cyclopentyloxy-4-methoxyphenyl)-2-phenylethenyl]-3,5-dichloropyridine;
2-[2-(3-Cyclopentyloxy-4-methoxyphenyl)-2-phenylethenyl] pyridine;
4-[1 (3-Cyclopentyloxy-4-methoxyphenyl)-2-(4-pyridyl) ethenyl] aniline;
4-[1-(3-Cyclopentyloxy-4-methoxyphenyl)-2-(4-pyridyl)ethenyl] benzoic acid;
Ethyl N-{4-[-(3-Cydopentyloxy-4-methoxyphenyl)-2-(4-pyridyl) ethenyl]phenyl)carbamate;
N-{4-[1 -(3-Cyclopentyloxy-4-methoxyphenyl)-2(4-pyridyl)ethenyl] phenyl}N'-ethylurea;
N-{4-[1-(3-Cyclopentyloxy-4-methoxyphenyl)]-2-(4-pyridyl)ethenyl} phenylacetamide;
3-[2-(3-Cyclopentyloxy-4-methoxyphenyl)-2-phenylethenyl] pyridine;
4-[2-2(3-Cyclopentyloxy-4-methoxyphenyl)-2-phenylethenyl] pyrimidine;
4-[2-(3-Cyclopentyloxy-4-methoxyphenyl)-2-(4-hydroxymethylphenyl)ethenyl]pyridine;
4-[1-(3-Cyclopentyloxy-4-methoxyphenyl)-2-(4-pyridyl)ethenyl] benzamide;
Ethyl-4-[1-(3-Cyclopentyloxy-4-methoxyphenyl)-2-(4-phenylethenyl]benzoate;
N-{4-[1-(3-Cyclopentyloxy-4-methoxyphenyl)-2-(4-pyridyl)ethenyl] phenyl)methanesulphonamide; or
each (E) or (Z) isomer thereof; and the salts, solvates, hydrates and N-oxides thereof.

Compounds according to the invention are selective and potent inhibitors of PDE IV. The ability of the compounds to act in this way may be simply determined by the tests described in the Examples hereinafter.

The compounds according to the invention are thus of particular use in the prophylaxis and treatment of human diseases where an unwanted inflammatory response or muscular spasm (for example bladder or alimentary smooth muscle spasm) is present and where the elevation of cAMP levels may be expected to prevent or alleviate the inflammation and relax muscle.

Particular uses to which the compounds of the invention may be put include the prophylaxis and treatment of asthma, especially inflamed lung associated with asthma, cystic fibrosis, or in the treatment of inflammatory airway disease, chronic bronchitis, eosinophilic granuloma, psoriasis and other benign and malignant proliferative skin diseases, endotoxic shock, septic shock, ulcerative colitis, Crohn's disease, reperfusion injury of the myocardium and brain, inflammatory arthritis, chronic glomerulonephritis, atopic dermatitis, urticaria, adult respiratory distress syndrome, diabetes insipidus, allergic rhinitis, allergic conjunctivitis, vernal conjunctivitis, arterial restenosis and artherosclerosis.

Compounds of the invention also suppress neurogenic inflammation through elevation of cAMP in sensory neurones. They are, therefore, analgesic, anti-tussive and anti-hyperalgesic in inflammatory diseases associated with irritation and pain.

Compounds according to the invention may also elevate cAMP in lymphocytes and thereby suppress unwanted lymphocyte activation in immune-based diseases such as rheumatoid arthritis, ankylosing spondylitis, transplant rejection and graft versus host disease.

Compounds according to the invention have also been found to reduce gastric acid secretion and therefore can be used to treat conditions associated with hypersecretion.

Compounds of the invention suppress cytokine synthesis by inflammatory cells in response to immune or infectious stimulation. They are, therefore, useful in the treatment of bacterial, fungal or viral induced sepsis and septic shock in which cytokines such as tumour necrosis factor (TNF) are key mediators. Also compounds of the invention suppress inflammation and pyrexia due to cytokines and are, therefore, useful in the treatment of inflammation and cytokine-mediated chronic tissue degeneration which occurs in diseases such as rheumatoid or osteo-arthritis.

Over-production of cytokines such as TNF in bacterial, fungal or viral infections or in diseases such as cancer, leads to cachexia and muscle wasting. Compounds of the invention ameliorate these symptoms with a consequent enhancement of quality of life.

Compounds of the invention also elevate cAMP in certain areas of the brain and thereby counteract depression and memory impairment.

Compounds of the invention suppress cell proliferation in certain tumour cells and can be used, therefore, to prevent tumour growth and invasion of normal tissues.

For the prophylaxis or treatment of disease the compounds according to the invention may be administered as pharmaceutical compositions, and according to a further aspect of the invention we provide a pharmaceutical composition which comprises a compound of formula (1) together with one or more pharmaceutically acceptable carriers, excipients or diluents.

Pharmaceutical compositions according to the invention may take a form suitable for oral, buccal, parenteral, nasal, topical or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds for formula (1) may be formulated for parenteral administration by injection e.g. by bolus injection or infusion. Formulations for injection may be presented in unit dosage form, e.g. in glass ampoule or multi dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use.

In addition to the formulations described above, the compounds of formula (1) may also be formulated as a depot preparation. Such long acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular inflammatory condition will vary depending on the compound chosen, and the condition of the patient to be treated. In general, however, daily dosages may range from around 100ng/kg to 100mg/kg, e.g. around 0.01mg/kg to 40mg/kg body weight for oral or buccal administration, from around 10ng/kg to 50mg/kg body weight for parenteral administration and around 0.05mg to around 1000mg e.g. around 0.5mg to around 1000mg for nasal administration or administration by inhalation or insufflation.

The compounds according to the invention may be prepared by the following processes. The symbols R¹, R², R³ and R⁴ when used in the formulae below are to be understood to represent those groups described above in relation to formula (1) unless otherwise indicated. In the reactions described below it may be necessary to protect reactive functional groups, for example hydroxy, amino, thio, or carboxy groups, where these are desired in the final product, to avoid their unwanted participation in the reactions. Conventional protecting groups may be used in accordance with standard practice [see, for example, Green, T. W. in "Protective Groups in Organic Synthesis". John Wiley and Sons, 1981.] It may be that deprotection will form the last step in the synthesis of compounds of formula (1). Thus, in one example, compounds of formula (1) wherein R³ and/or R⁴ contains a carboxylic acid group may be prepared by deprotecting the corresponding compound wherein R³ and/or R⁴ contains a protected carboxyl group, such as an oxazolinyl group, e.g. 4,4-dimethyl-2-oxazolinyl, in the presence of a base, e.g. sodium hydroxide, in an acid solvent e.g. aqueous hydrochloric acid, at an elevated temperature, e.g. the reflux temperature.

Thus according to a further aspect of the invention, a compound of formula (1) may be prepared by dehydration of an alcohol of formula (3): 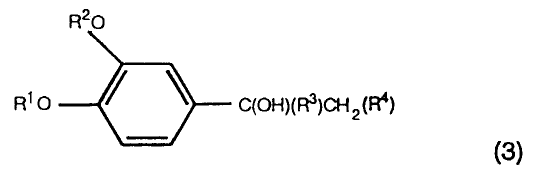 using an acid or base-catalysed elimination.

Suitable acids include for example phosphoric or sulphonic acids, e.g. 4-toluenesulphonic acid. The reaction may be performed in an inert organic solvent, for example a hydrocarbon such as toluene, at an elevated temperature, for example the reflux temperature. Base catalysed elimination may be performed using for example trifluoroacetic anhydride in the presence of an organic base such as triethylamine at a low temperature e.g. from around 0°C to ambient temperature, in a solvent such as dichloromethane or tetrahydroduran.

In certain instances, the reaction conditions used may also cleave the group R² in the starting material of formula (4) to yield a compound of formula (1) where R² is a hydrogen atom. Such compounds may be converted to the required compound of formula (3) by a further process according to the invention using a halide R²Hal (where Hal is a halogen atom such as a bromine or chlorine atom) where necessary in the presence of a base such as caesium or potassium carbonate or an alkoxide such as potassium t-butoxide, in a dipolar aprotic solvent such as an amide, e.g. a substituted amide such as dimethylformamide at ambient temperature or above e.g. around 40°C to 50°C.

Intermediates of formula (3) may be prepared by reaction of a ketone of formula (4): 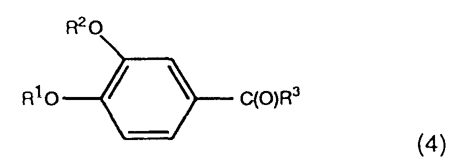 with an organometallic reagent R⁴CH₂Z where Z is a metal atom.

Metal atoms represented by Z include, for example, a lithium atom.

The reaction may be performed in a solvent such as an ether, e.g. a cyclic ether such as tetrahydrofuran, at a low temperature, e.g. around -70°C to ambient temperature. This reaction is particularly suitable for the preparation of compounds of formula (1) wherein R⁴ is an electron deficient group such as a 2- or 4-pyridyl group.

Reagents R⁴CH₂Z are either known compounds or may be prepared, preferably in situ during the above process, by reaction of a compound AlkCH₂Z [where Alk is an alkyl group such as a n-propyl group] with a compound R⁴CH₃ where necessary in the presence of a base such as an amine e.g. diisopropylamine using the above-mentioned conditions.

Ketones of formula (4) may be prepared by oxidation of a corresponding alcohol of formula (5): 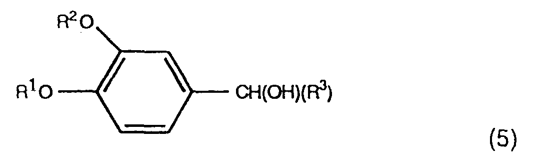 using an oxidising agent such as manganese dioxide in a solvent such as dichloromethane at ambient temperature.

Alternatively, ketones of formula (4) may be prepared by reaction of a halide of formula (6): 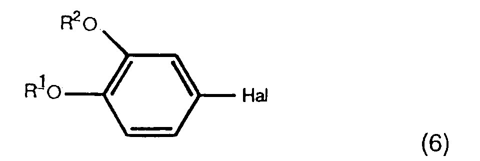 [where Hal is a halogen atom such as a bromine or chlorine atom] by halogen-metal exchange with a base such as n-butyllithium, followed by reaction with a nitrile R³CN, an acid chloride R³COCI or an ester R³CO₂Alk (where Alk is an alkyl group, e.g. a methyl group) in a solvent such as tetrahydrofuran, at a low temperature, e.g. around -70°C, and subsequent treatment with an acid such as hydrochloric acid at e.g. -20°C to ambient temperature.

Alcohols of formula (5) may be prepared by reaction of an aldehyde of formula (7): 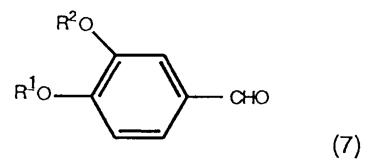 with organometallic compound, such as an organolithium compound R³Li, or a Grignard reagent R³MgBr, in a solvent, such as tetrahydrofuran ,at a low temperature e.g. around -55°C to 0°C.

Aldehydes of formula (7) may be prepared by alkylation of a corresponding compound of formula (8): 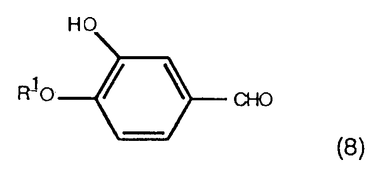 using a compound R²Hal [where Hal is as previously defined] using the reagents and conditions described herein above for the alkylation of intermediates of formula 4.

Intermediates of formula (8) are either known compounds or may be prepared from known starting materials by methods analogous to those used for the preparation of the known compounds.

Halides of formula (6) may be prepared by alkylation of a compound of formula (9): 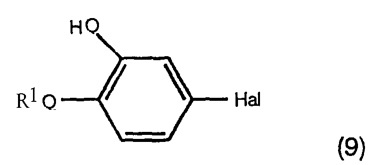 using the reagents and conditions discussed above in relation to the alkylation of aldehydes of formula (8).

Halides of formula (9) may be prepared by oxidation of an aldehyde of formula (10): 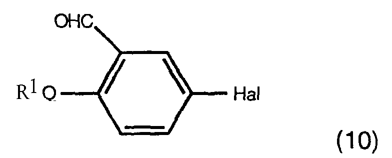 using an oxidising agent such as 3-chloroperoxybenzoic acid in a halogenated hydrocarbon such as chloroform at a temperature from around 0°C to room temperature.

In yet another aspect of the invention, compounds of formula (1) may be obtained by coupling a compound of formula (11) 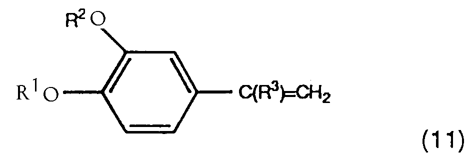 in a Heck reaction with an organopalladium compound derived from a compound R⁴Hal [where Hal is a halogen atom, such as a bromine atom] and a palladium salt, such as palladium acetate, in the presence of a phosphine such as tri-o-tolylphosphine and a base such as triethylamine at an elevated temperature and pressure.

Intermediate alkenes of formula (11) may be obtained by reaction of a corresponding ketone of formula (4) (described herein above) using a Wittig reaction employing a phosphonium salt such as methyltriphenylphosphonium bromide in the presence of a base such as n-butyllithium and in inert solvent such as tetrhydrofuran at, for example, 0°C to ambient temperature.

In a further process according to the invention a compound of formula (1) may be prepared by dehydrogenation of a compound of formula (12): 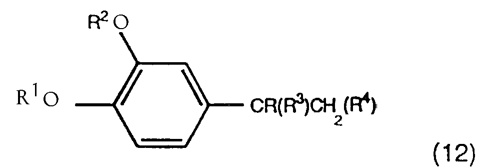 where R is a hydrogen atom,
using a dehydrogenating reagent.

Suitable dehydrogenating reagents include for example quinones such as 2,3-dichloro-5,6-dicyano-1,4-benzoquinone, in a solvent, e.g. dioxane, at an elevated temperature, e.g. the reflux temperature.

Compounds of formula (12) may be prepared by cyclisation of a compound of formula (13) 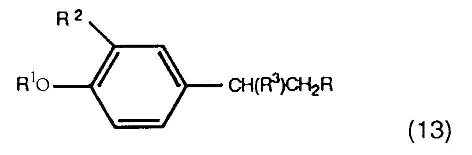 where R is a carboxylic acid [-CO₂H] group or a reactive derivative thereof; or a nitrile [-CN] or an imine salt with a bifunctional reagent W¹R^{4a}W² and, where necessary, a compound R^{4b}W³ [where W¹, W² and W³, which may be the same or different, is each a reactive functional group or a protected derivative thereof; and R^{4a} and R^{4b} are components of the heteroaryl group R⁴ such that when added together with W¹, W² and W³ to the group R in compounds of formula (15) the resulting group -RW¹ R^{4a}W² or -RW¹R^{4a}W²R^{4b}W³ constitutes the heteroaryl group R⁴].

Reactive derivatives of carboxylic acids for use in this reaction include acid halides, (e.g. acid chlorides), amides, including thioamides, or esters, including thioesters. Imine salts include for example salts of formula [e.g.- C(OAlk)=NH₂⁺A⁻, where Alk is a C₁₋₄alkyl group and A- is a counterion e.g. a chloride ion].

In this general reaction the reactive functional groups represented by W¹, W² or W³ may be any suitable carbon, nitrogen, sulphur or oxygen nucleophiles. Particular examples include simple nucleophiles such as carbanions [e.g. generated by the coupling of an alkyl group with an organometallic compound], amino, thiol and hydroxyl groups.

In general, the cyclisation reaction will initially be performed in a solvent, for example an inert solvent such as a halocarbon, e.g. dichloromethane, an ether, e.g. a cyclic ether such as tetrahydrofuran, or a hydrocarbon, e.g. an aromatic hydrocarbon such as toluene, from a low temperature, e.g. around -70°C, to around the reflux temperature, where necessary in the presence of a base or a thiation reagent, e.g. Lawesson's reagent, followed if necessary by heating, to an elevated temperature, e.g. the reflux temperature.

In an example of the general cyclisation process, a compound of formula (13) where R is an acid halide as described above may be reacted with a compound W¹R^{4a}W² which is a monoalkylmalonate, e.g. ethyl hydrogen malonate, followed by reaction with a compound R^{4b}W³ which is hydrazine to give a compound of formula (1) wherein R⁴ is a 5-hydroxypyrazolyl group.

Intermediate compounds of formula (13) are particularly useful and form a further aspect of the invention. Active derivatives of the acids of formula (13) and other compounds of formula (13) where R is a nitrile or an imine salt may be prepared from the corresponding acids [where R is -CO₂H] using conventional procedures for converting carboxylic acids to such compounds, for example as described in the Examples hereinafter.

Acids of formula (13) [where R is -CO₂H] may be prepared by hydrolysing a diester of formula (14) 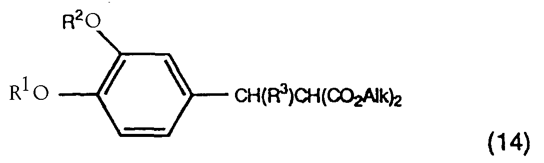 where Alk is a C₁₋₄alkyl group, e.g. an ethyl group, with a base, e.g. sodium hydroxide, in a solvent, e.g. dioxane, at an elevated temperature, e.g. the reflux temperature, followed by acidification at an elevated temperature.

Diesters of formula (14) may be prepared by reacting a diester of formula (15) 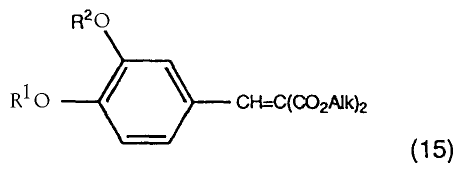 with an organometallic reagent, such as a Grignard reagent using the conditions described above for the preparation of alcohols of formula (3) (where R is a hydroxy group).

In another process according to the invention, a compound of formula (12) may be prepared by alkylation of a compound of formula (16): 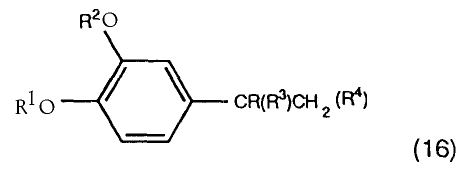 using a reagent R²L, where L is a leaving group.

Leaving groups represented by L include halogen atoms such as iodine or chlorine or bromine atoms or sulphonyloxy groups such as arylsulphonyloxy groups, e.g. p-toluenesulphonyloxy.

The alkylation reaction may be carried out in the presence of a base, e.g. an inorganic base such as a carbonate, e.g. caesium or potassium carbonate, an alkoxide, e.g. potassium-t-butoxide, or a hydride, e.g. sodium hydride, in a dipolar aprotic solvent such as an amide, e.g. a substituted amide such as dimethylformamide or an ether, e.g. a cyclic ether such as tetrahydrofuran, at ambient temperature or above e.g. around 40°C to 50°C.

Intermediates of formula (16) may be obtained from the corresponding protected compound of formula (17): 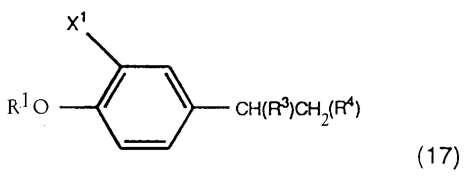 wherein X¹ is a protected hydroxy, thio or amino group using conventional procedures [see Green, T. W. *ibid*]. Thus, for example, where X' is a t-butyldimethylsilyloxy group, the required hydroxyl group may be obtained by treatment of the protected intermediate with tetrabutylammonium fluoride. The protected intermediate of formula (16) may be prepared in an analogous manner to the compounds of formula (1) using the reactions described herein and appropriately protected intermediates.

Compounds of formula (15) may be prepared by condensing an aldehyde of formula (7) with a malonate, e.g. diethylmalonate, if necessary in the presence of catalysts, e.g. piperidine and acetic acid, in an inert solvent, e.g. toluene, at elevated temperature, e.g. the reflux temperature.

Compounds of formula (1) may also be prepared by interconversion of other compounds of formula (1). Thus, for example, a group represented by R³ or R⁴ in compounds of formula (1) may be substituted in the aryl or heteroaryl portions by an appropriate substitution reaction using the corresponding unsubstituted compound of formula (1) and a substituent containing nucleophile or electrophile.

In another example of an interconversion process a compound of formula (1) wherein the aryl or heteroaryl group in R³ or R⁴ contains a -CH₂NH₂ substituent may be prepared by reduction of a corresponding compound wherein R⁵ contains a nitrile group, using for example a complex metal hydride such as lithium aluminium hydride in a solvent such as an ether e.g. diethylether.

In a further example, a compound of formula (1) wherein the aryl or heteroaryl group in R³ and/or R⁴ contains an alkanoylamino or alkanoylaminoalkyl substituent may be prepared by acylation of a corresponding compound wherein R³ and/or R⁴ contains a -NH₂ or alkylamino group by reaction with an acyl halide in the presence of a base, such as a tertiary amine e.g. triethylamine in a solvent such as dichloromethane.

In yet another example of an interconversion process, compounds of formula (1) wherein R³ and/or R⁴ is substituted by an ester [CO₂Alk²], e.g. an ethanoate, may be prepared by esterification of a corresponding compound wherein R³ and/or R⁴ contains a carboxylic acid, using an acid halide, such as an acid chloride, e.g. acetyl chloride, in an alcohol, such as ethanol, at an elevated temperature, such as the reflux temperature.

Compounds of formula (1) wherein R³ and/or R⁴ is substituted by a carboxylic acid may be prepared from the corresponding compound wherein R³ and/or R⁴ contains a formyl group, by oxidation with an oxidising agent, e.g. potassium permanganate, in a solvent, such as an alcohol, e.g. tert-butanol, at ambient temperature.

In a further interconversion reaction, compounds of formula (1) wherein R³ and/or R⁴ is substituted by an aminoalkyl group, such as dimethylaminomethyl, may be prepared by reductive amination of a corresponding compound wherein R³ and/or R⁴ contains a formyl group, using an amine, e.g. dimethylamine, in the presence of a reducing agent, e.g. sodium cyanborohydride, if necessary in the presence of a catalyst, e.g. ethanolic HCI, in a solvent, such as an alcohol, e.g. methanol, at ambient temperature.

In another example of an interconversion reaction a compound of formula (1) wherein R³ and/or R⁴ is substituted by a formyl group, may be reduced to the corresponding alcohol, e.g. where R³ and/or R⁴ contains a hydroxymethyl group, using a reducing agent, e.g. sodium borohydride, in a solvent, such as an alcohol, e.g. ethanol, at a temperature from around 20°C to ambient temperature. The resulting alcohol may then be converted to the corresponding alkoxy derivative, e.g. methoxymethyl, by reaction with an alkyl halide or alkyl sulphonate using the methods and reagents described above for the alkylation of intermediates of formula (18).

In a further example of an interconversion process compounds of formula (1) wherein R³ and/or R⁴ contains a carboxamido (-CONHR¹¹) or an aminocarbonyl (-NHCOR¹¹) group may be prepared by reaction of the corresponding compound wherein R³ and/or R⁴ contains a -CO₂H or a -NH₂ group respectively by reaction with a carbamate, such as isobutyl chloroformate or ethyl chloroformate, in the presence of a base, such as an amine, e.g. triethylamine or N-methylmorpholine, in a solvent, such as dichloromethane, or a mixture of solvents, e.g. tetrahydrofuran and dimethylformamide, at a temperature from around -20°C to room temperature.

N-oxides of compounds of formula (1) may be prepared by oxidation of the corresponding nitrogen base using an oxidising agent such as hydrogen peroxide in the presence of an acid such as acetic acid, at an elevated temperature, for example around 70°C to 80°C, or alternatively by reaction with a peracid such as peracetic acid in a solvent, e.g. dichloromethane, at ambient temperature.

Salts of compounds of formula (1) may be prepared by reaction of a compound of formula (1) with an appropriate acid or base in a suitable solvent e.g. an organic solvent such as an ether using conventional procedures.

The following examples illustrate the invention. The following abbreviations are used: DMF - dimethylformamide; THF - tetrahydrofuran; DME - dimethoxyethane; EtOAc - ethyl acetate; Et₂O - diethylether; Et₃N - triethylamine; CH₂Cl₂ - dichloromethane; BuLi - butyllithium; LDA - lithium diisopropylamide; EtOH - ethanol; RT - room temperature.

### INTERMEDIATE 1

### 3-Cyclopentyloxy-4-methoxybenzaldehyde

Cs₂CO₃ (214g, 0.66mol) was added to a mixture of 3-hydroxy-4-methoxybenzaldehyde (100g, 0.66mol) and cyclopentyl bromide (98g, 0.66mol) in anhydrous DMF (500ml). The reaction mixture was stirred at RT for 16h then treated with a further portion of cyclopentyl bromide (98g, 0.66mol) and Cs₂CO₃ (214g, 0.66mol). After a further 6h at RT, the mixture was filtered and concentrated *in vacuo.* The residue was dissolved in CH₂Cl₂ (300ml) and washed with NaOH solution (10%; 2x150ml). The organic layer was dried (MgSO₄), concentrated *in vacuo* and distilled (150°C, 10⁻²mbar) to afford the title compound (130g) as a viscous colourless oil. δ_{H} (CDCl₃) 1.5-2.0 (8H, br m, CH₂)₄), 3.87 (3H, s, OMe), 4.80 (1H, br m, OCHCH₂), 6.90 (1H, d, J 8.7Hz, ArH ortho to OMe), 7.30-7.45 (2H, m, 2xArH meta to OMe), and 9.77 (1H, s, ArCHO).

### INTERMEDIATE 2

a) **(3-Cyclopentyloxy-4-methoxyphenyl)phenylketone**
   Phenyllithium (1.5M in ether-cyclohexane, 33.5ml, 50mmol) was added dropwise to a solution of Intermediate 1 (10.0g, 45.4mmol) in THF (50ml) at about -55°C. The reaction mixture was allowed to warm to RT overnight then diluted with water (100ml) and extracted with Et₂O (3x50ml). The organic extract was washed with aqueous HCI (1%, 70ml), brine (100ml), then dried (MgSO₄), and concentrated *in vacuo* to afford 1 (3-cyclopentyloxy-4-methoxyphenyl)-1-phenylmethanol (13.4g) as a white solid. m.p. 82.5-83°C; δ_{H} (CDCl₃) 1.5-2.0 (8H, br, m, (CH₂)₄), 2.30 (1H, br, s, OH), 3.77 (3H, s, OMe), 4.68 (1H, br, m, OCHCH₂), 5.77 (1H, s, CHOH), 6.75-6.85 (3H, m, ArH ortho to OMe + 2xArH meta to OMe), and 7.15-7.4 (5H, m, C₆H₅); m/z 298 (M⁺ 20%), 230 (50), 151 (30), 125 (100), 124 (33), 105 (38), and 92 (22).
   The alcohol (prepared above) (13.4g, 44.8mmol) was dissolved in CH₂Cl₂ (150ml) and treated with MnO₂ (22g). The reaction mixture was vigorously stirred at RT for 18h then treated with a further portion of MnO₂ (20g). More MnO₂ (20g) was added after 10h and the mixture stirred for 18h then filtered through Celite® and concentrated *in vacuo.* The residue was recrystallised from EtOH to afford the title compound (11.27g; two crops) as a white crystalline solid m.p. 59-75°C; δ_{H} (CDCl₃) 1.5-2.1 (8H, br, m, (CH₂)₄), 3.88 (3H, s, OMe), 4.80 (1H, br m, OCHCH₂), 6.83 (1H, d, J 8.5 Hz, ArH ortho to OMe), and 7.25-7.8 (7H, m, 2xArH meta to OMe + C₆H₅); m/z 296 (M⁺ 11%), 229 (17), 228 (95), 152 (12), 151 (100), 105 (30), 77 (21), and 41(10).
   The following intermediate was prepared in a manner similar to Intermediate 2a.
b) **(3-Cyclopentyloxy-4-methoxyphenyl)(2-methoxyphenyl)ketone** From Intermediate 4 (1.35g, 5.0mmol) and 2-methoxybenzaldehyde (0.68g, 5.0mmol). Chromatography (SiO₂; EtOAc/hexane, 1:1) afforded the title compound (1.439) as a white solid (Found: C, 73.53, H, 6.86. C₂₀H₂₂O₄ requires C, 73.60; H, 6.79%); m/z (El) 326 (M⁺, 28%), 258 (65), 241 (82), 151 (67), 138 (32), 135 (100), and 121 (45).

### INTERMEDIATE 3

### 5-Bromo-2-methoxyphenol

A solution of 5-bromo-2-methoxybenzaldehyde (100g, 0.46mol) in CHCl₃ (250ml) was cooled with an ice bath and 3-chloroperoxybenzoic acid (50-60% purity) (146g, 0.51mol) in CHCl₃ (1000ml) added. The reaction mixture was allowed to warm slowly to room temperature and stirred for 72h. The white solid was filtered off and the filtrate concentrated *in vacuo.* The residue was dissolved in Et₂O (200ml) and washed with 1M sodium sulphite solution (2x200ml) then NaHCO₃ [half saturated] (3x200ml). The ether layer was washed with 10% aqueous NaOH (3x100ml) and the combined basic extract was acidified with concentrated hydrochloric acid and extracted with Et₂O (3x100ml). The combined organic extract was dried (MgSO₄) and florisil (10g) filtered and the solvent removed under reduced pressure to give the title compound (90g) as a pale brown solid.

### INTERMEDIATE 4

### 4-Bromo-2-cyclopentyloxyanisole

Intermediate 3 (90g)was dissolved in DMF (300ml), and treated with Cs₂CO₃ (158g, 490mmol), and cyclopentyl bromide (73g, 52.5ml, 490mmol). After stirring overnight, further Cs₂CO₃ (35g, 107mmol) and cyclopentylbromide (12ml, 16.7g, 112mmol) were added and stirring continued for 2h. Further portions of cyclopentylbromide (10ml) and Cs₂CO₃ were then added (14g). After stirring for 1h, the DMF was evaporated *in vacuo* and the residue diluted with water (200 ml) and extracted with Et₂O (3x100ml). The combined organic extract was washed with NaOH solution (5%, 2x100ml), water (100ml), then dried (MgSO₄) and the solvent evaporated *in vacuo* to give a red oil which was distilled (140°C, 0.3mbar) to afford the title compound (101g) as a colourless oil (Found: C, 53.11; H, 5.53. C₁₂H₁₅BrO₂ requires C, 53.15; H, 5.58%).

### INTERMEDIATE 5

### (3-Cyclopentyloxy-4-methoxyphenyl)(4-pyridyl)ketone

n-BuLi (1.45M in hexanes; 19.6ml, 28.4mmol) was added dropwise at -70°C to a solution of Intermediate 4(7.0g, 25.8mmol) in THF (50ml). After stirring for 0.25h, a solution of 4-cyanopyridine (3.08g, 29.7mmol) in THF (15ml) was added and maintained at -70°C for 0.75h. The reaction mixture was then allowed to warm to -10°C and quenched with aqueous HCI (10%; 60ml). The mixture was stirred for 0.5h, basified with aqueous NaOH (10%, 70ml), and extracted with Et₂O (3x70ml). The extract was washed with brine (100ml), dried (MgSO₄), and concentrated *in vacuo.* The residue was subjected to chromatography (SiO₂; EtOAc/hexane, 4:1) to afford the title compound (6.34g) as a white powder. δ_{H} (CDCl₃) 1.5-1.9 (8H, br m, (CH₂)₄), 3.90 (3H, s, OMe), 4.82 (1H, br m, OCHCH₂), 6.84 (1H, d, J 8.4 Hz, ArH ortho to OMe) 7.29 (1H, dd, J 8.4, 2.0 Hz, ArH para to cyclopentyloxy), 7.4-7.55 (3H, m, ArH ortho to cyclopentyloxy + pyridine H₃, H₅), and 8.73 (2H, dd, J 4.4 Hz, 1.5 Hz, pyridine H₂, H₆).

### INTERMEDIATE 6

### (E) and (Z) isomers of 4-[1-(3-Hydroxy-4-methoxyphenyl)-2-(4-pyridyl) ethenyl]pyridine

Intermediate 8 (0.72g, 1.85mmol) in toluene (120ml) containing 4toluenesulphonic acid (0.88g, 4.6mmol) was heated to reflux in a Dean-Stark apparatus for 18h. The cooled reaction mixture was treated with aqueous NaOH (10%) then taken to pH 7 with concentrated hydrochloric acid. The mixture was extracted with CH₂Cl₂ (3x40ml), the extract washed with saturated NaHCO₃ (100ml), and Na₂CO₃ (10%;2x60ml), then dried (MgSO₄), and concentrated *in vacuo* to afford the title compound (0.4g) as a yellow foam; δ_{H} (CDCl₃) (major isomer) 3.88 (3H, s, OMe), 6.6-6.9 (6H, m, ArH ortho to OMe + 2xArH meta to OMe + C=CH + pyridine H₃, H₅), 7.08 (2H, dd, J 4.6, 1.6 Hz, pyridine H₃, H₅), 8.30 (2H, dd, J 4.5, 1.6 Hz, pyridine H₂, H₆), and 8.51 (2H, dd, J 4.4, 1.6 Hz, pyridine H₂, H₆), [the minor isomer displays a signal at δ 3.90 (3H, s, OMe)].

### INTERMEDIATE 7

a) **(±)-4-[2-(3-Cyclopentyloxy-4-methoxyphenyl)-2-hydroxy-2-phenylethyl] pyridine**
   n-Buli (1.4M in hexanes; 2.7ml, 3.7mmol) was added dropwise at -70°C to a solution of 4-methylpyridine (0.35g, 3.72mmol) in THF (20ml). After 0.5h, a solution of Intermediate 2 (1.00g, 3.38mmol) in THF (4ml) was added over 5 min at -70°C, the mixture stirred for 1h at this temperature then allowed to warm to RT over 2h. The reaction mixture was partitioned between Et₂O (50ml) and water (50ml) and the organic layer was separated. The aqueous layer was further extracted with Et₂O (2x40ml) and the combined organic extract was dried (MgSO₄) and concentrated in *vacuo.* The residue was subjected to chromatography (SiO₂; EtOAc-hexane) to afford, first, Intermediate 2 (300mg) then the title compound (738mg) as a white solid. m.p. 148-149°C (toluene-hexane) (Found : C, 77.32; H, 7.04; N, 3.50. C₂₅H₂₇O₃ requires C, 77.09; H, 6.99; N, 3.60%); δ_{H} (CDCl₃) 1.4-1.9 (8H, br, m, (CH₂)₄), 2.3 (1H, v.br.s, OH exchanges with D₂O), 3.51 (2H, s, CH₂ pyridine), 3.78 (3H, s, OMe), 4.60 (1H, br, m, OCHCH₂), 6.65-6.9 (5H, m) and 7.15-7.4 (5H, m) (ArH ortho to OMe + 2xArH meta to OMe + C₆H₅+ pyridine H₃, H₅), and 8.22 (2H, dm, J 4.5Hz, pyridine H₂, H₆); m/z 389 (M⁺ 3%), 298 (15), 297 (69), 229 (27), 228 (37), 151 (43), 105 (100), 93 (52), 77 (24), and 41 (14).
   The following compounds were prepared in a manner similar to Intermediate 7a.
b) **(±)-2-[2-(3-Cyclogentyloxy-4-methoxyphenyl)-2-hydroxy-2-phenylethyl] pyrazine**
   From 2-methylpyrazine (1.0ml, 110mmol) and Intermediate 2 (3.24g, 11.0mmol). Trituration with Et₂O gave the title compound (0.885g) as a white solid. δ_{H} (CDCl₃) 1.45-1.9 (8H, br, m, (CH₂)₄), 3.73 (2H, s, CH₂ pyrazine), 3.80 (3H, s, OMe), 4.68 (1H, br, m, OCH), 6.22 (1H, br s, OH), 6.73 (1H, d, J 8.4 Hz, ArH ortho to OMe), 6.89 (1H, dd, J 8.4, 2.0Hz, ArH para to cyclopentyloxy), 7.0 (1H, d, J 2.0Hz, ArH ortho to cyclopentyloxy, 7.1-7.5 (5H, m, C₆H₅), and 8.37 (3H, s, pyrazine H₃, H₅ H₆).
c) **(±)-4-[2-(3-Cyclopentyloxy-4-methoxyphenyl)2-hydroxy-phenylethyl]-3,5-dichloropyridine**
   From Intermediate 11 (2.0g, 12.3mmol) and Intermediate 2 (3.65g, 12.3mmol). Purification by column chromatography (SiO₂;0-2% MeOH/ CH₂Cl₂) afforded the title compound (1.74g) as a white solid. m.p. 129-130°C. δ_{H} (CDCl₃) 1.5-1.9 (8H, br, m, (CH₂)₄), 2.65 (1H, br s, OH), 3.85 (3H, s, OMe), 3.92 (1H, d, J 14Hz, CH_{A}H_{B} pyridine), 3.98 (1H, d, J 14 Hz, CH_{A}H_{B} pyridine), 4.57 (1H, br, m, OCH), 6.7-6.9 (3H, m, ArH ortho + 2x ArH meta to OMe), 7.2-7.4 (5H, m, C₆H₅), and 8.36 (2H, s, pyridine H₂, H₆).
d) **4-[2-(4-Bromophenyl)2-(3-cyclopentyloxy-4-methoxyphenyl)-2-hydroxyethyl]pyridine**
   From 4-picoline (2.0ml, 1.90g, 20.4mmol) and Intermediate 26 (7.30g, 19.5mmol). Purification by column chromatography (SiO₂; gradient elution 50-75%, EtOAc/hexane) gave the title compound (7.77g) as a pale yellow foamy solid. Found: C, 63.82; H, 5.58; N, 2.96. C₂₅H₂₆BrNO₃ requires C, 64.11; H, 5.60; N, 2.99%, δ_{H} (CDCl₃) 1.5-1.9 (8H, br, m, (CH₂)₄), 2.7 (1H, br s, OH), 3.46 (1H, d, J 13.1Hz, CH_{A}H_{B} pyridine), 3.54 (1H, d, J 13.1 Hz, CH_{A}H_{B} pyridine), 3.82 (3H, s, OMe), 4.64 (1H, br m, OCH), 6.75-6.9 (5H, m, C₆H₃ + pyridine H₃, H₅), 7.21 (2H, ca. d, J 8.7Hz, ArH of C₆H₄), and 8.29 (2H, ca. d, J 6.0Hz, pyridine H₂, H₆) ν_{max.} (CDCl₃) 3604, 1605, 1513, and 1256 cm⁻¹ ; m/z (ESI) 470 (M⁺ +2, 20%), 468 (M⁺, 18), 377 (52), 375 (55), 95 (13), and 94 (100).
e) **(±)-4-{2-(3-Cyclopentyloxy-4-methoxyphenyl)-2-[4-(4,4-dimethyl-2-oxazolinyl)phenyl]-2-hydroxyethyl)pyridine**
   From 4-methylpyridine (1.45g, 1.52ml, 15.6mmol) and Intermediate 14 (5.82g, 14.9mmol). Trituration with Et₂O gave the title comound (6.61g) as an off-white solid. δ_{H} (CDCl₃) 1.37 (6H, s, CMe), 1.55-1.8 (8H, m, (CH₂)₄), 2.7 (1H, v. br s, OH), 3.56 (2H, br s, CH₂ pyridine), 3.82 (3H, s, OMe), 4.10 (2H, s, oxazoline CH₂), 4.63 (1H, m, OCH), 6.75-6.9 (5H, m, ArH), 7.37 (2H, d, J 8.6Hz, pyridine H₃, H₅), 7.85 (2H, d, J 7.3Hz, ArH ortho to oxazoline) and 8.29 (2H, br s, pyridine H₂, H₆); ν_{max.} (CDCl₃) 3603, 1649, 1512, and 1257 cm⁻¹ ; m/z (ESI) 487 (M⁺ +1, 100%), and 394 (61).

### INTERMEDIATE 8

### (±)-4-[1-(3-Cyclopentyloxy-4-methoxyphenyl)-1-hydroxy-2-(4-pyridyl) ethyl]pyridine

n-BuLi (1.45M in hexanes; 5.1ml,7.41mmol) was added dropwise at -70°C to a solution of 4-methylpyridine (0.69g, 7.41mmol) in THF (20ml). After 0.5h a solution of Intermediate 5 (2.0g, 6.73mmol) in THF (10ml) was added dropwise over 5 min. The reaction mixture was stirred for 0.5h at -70°C then at RT for 0.5h. Water (50ml) was added and the mixture extracted with EtOAc (3x60ml). The extract was washed with brine (80ml), dried (MgSO₄), and concentrated *in vacuo.* The residue was subjected to chromatography (SiO₂; EtOAc to EtOAc/CH₃OH, 9:1) to afford the title compound (2.33g) as a white amorphous solid m.p. 99-103°C; δ_{H} (CDCl₃) 1.5-2.0 (9H, br, m, (CH₂)₄ + OH), 3.49 (2H, d, J 2.3 Hz, CH₂ COH), 4.65 (1H, br m, OCHCH₂), 6.7-6.9 (5H, m, ArH ortho to OMe+ 2xArH meta to OMe + pyridine H₃, H₅), 7.20 (2H, dd, J 4.6, 1.6 Hz, pyridine H₃, H₅), 8.22 (2H, dd, J 4.6, 1.6 Hz, pyridine H₂, H₆), and 8.40 (2H, dd, J 4.6, 1.6 Hz, pyridine H₂, H₆); m/z 390 (M⁺ 3%), 298 (21), 297 (14), 230 (21), 229 (91), 151 (100), 106 (22), 93 (27), 78 (12), and 41 (23).

### INTERMEDIATE 9

### 1-(3-Cyclopentyloxy-4-methoxyphenyl)-1-phenylethene

To a cold suspension (0°C) of methyl triphenylphosphonium bromide (53.6g; 0.15mol) in THF (500ml) under a nitrogen atmosphere was added n-BuLi (1.6M in hexanes; 94ml, 0.15mol) dropwise and the reaction mixture stirred at 0°C for 1h. A solution of Intermediate 2 (29.6g, 0.1mol) in THF (100ml) was added dropwise and the stirred reaction mixture allowed to warm to RT over 3h. The mixture was poured into 10% NH₄Cl solution (600ml) and extracted with CH₂Cl₂ (2x500ml). The combined organic layer was dried (MgSO₄), filtered and concentrated *in vacuo.* The residual slurry was triturated with hot hexane (500ml), the precipitated phosphine oxide filtered off and the filtrate evaporated *in vacuo* to yield the title compound (28.85g) as a yellow oil. δ_{H} (CDCl₃) 1.5-2.0 (8H, br m, (CH₂)₄), 3.85 (3H, s, OMe), 4.71 (1H, br m, OCH), 5.38 (2H, dd, J 10.5, 1.3Hz, C=CH₂), 6.75-6.9 (3H, m, C₆H₃), and 7.3-7.5 (5H, m, C₆H₅).

### INTERMEDIATE 10

a) **(±)-3-[2-(3-Cyclopentyloxy-4-methoxyphenyl)-2-hydroxy-2-phenylethyl]-2-methoxypyrazine**
   n-BuLi (1.6M in hexanes; 6ml, 12mmol) was added dropwise at 4°C to a solution of N, N-diisopropylamine (1.85ml, 13mmol) in THF (40ml). After 0.5h, 2-methoxy-3-methylpyrazine (1.28ml, 11mmol) was added dropwise at -70°C and the mixture stirred for 2h at this temperature. A solution of Intermediate 2 (3.26g, 11mmol) in THF (20ml) was added over 10 min at -70°C and the mixture stirred for a further 1h and then allowed to warm to RT. The reaction mixture was partitioned between CH₂Cl₂ (75ml) and saturated NaHCO₃ (100ml). The organic layer was separated, combined with further CH₂Cl₂ extracts (2x75ml), dried (MgSO₄) and concentrated *in vacuo.* The residue was subjected to chromatography (SiO₂; CH₂Cl₂) to afford the title compound (2.94g) as a white foam. δ_{H} (CDCl₃) 1.5-2.0 (8H, br m, (CH₂)₄), 3.63 (1H, d, J 14 Hz, CHH pyrazine), 3.77 (1H, d, J 14Hz, CHH pyrazine), 3.79 (3H, s, OMe ortho to cyclopentyloxy), 3.97 (3H, s, pyrazine OMe), 4.67 (1H, br m, OCH), 6.72 (1H, dd, J 8.4Hz, ArH ortho to OMe), 6.77 (1H, s, OH), 6.91 (1H, dd, J 8.4Hz, 2.0Hz, ArH para to cyclopentyloxy), 7.00 (1H, d, J 2.0Hz, ArH ortho to cyclopentyloxy), 7.1-7.5 (5H, m, C₆H₅), and 7.85-7.95 (2H, m, pyrazine H₅, H₆).
b) **(±)-2-[-2-(3-Cyclopentyloxy-4-methoxyphenyl)-2-hydroxy-2-phenylethyl]-4-methylpyridine**
   From 2,4-dimethylpyridine (1.7ml, 14.5mmol) and Intermediate 2 (4.30g, 14.5mmol). Purification by chromatography (SiO₂; CH₂Cl₂) afforded the title compound (1.23g) as a colourless oil (Found: C, 77.07; H, 7.10; N, 3.25. C₂₆H₂₉NO₃ requires C, 77.39; H, 7.24; N, 3.47%); δ_{H} (CDCl₃) 1.4-1.9 (8H, br m, (CH₂)₄), 2.25 (3H, s, pyridine Me), 3.60 (2H, s, CH₂ pyridine), 3.77 (3H, s, OMe), 4.68 (1H, br m, OCH), 6.72 (1H, d, J 8.5Hz, ArH ortho to OMe), 6.8-6.95 (3H, m, ArH para to cyclopentyloxy + pyridine H₃, H₅), 7.02 (1H, d, J 2.2Hz, ArH ortho to cyclopentyloxy), 7.1-7.3 (3H, m, meta and para ArH of C6H5), 7.46 (2H, ca d, J 8.5Hz, ortho ArH of C₆H₅), and 8.23 (1H, ca d, J 6 Hz, pyridine H₆); m/z (ESI) 404 (M⁺+1, 72%), 387 (13), and 386 (100).
c) **(±)-4-[2-(3-Cyclopentyloxy-4-methoxyphenyl)-2-hydroxy-2-phenylethyl]pyrimidine**
   From 4-methylpyrimidine (1.0ml) and Intermediate 2 (3.98g). Purification by chromatography (SiO₂;CH₂Cl₂) afforded the title compound (2.56g) as a white solid; δ_{H} (CDCl₃) 1.5-2.0 (8H, br m, (CH₂)₄), 3.66 (2H, s, CH₂ pyrimidine), 3.77 (3H, s, OMe), 4.65 (1H, br m, OCH), 6.58 (1H, s, OH), 6.72 (1H, d, J 8.4Hz, ArH ortho to OMe), 6.85 (1H, dd, J 8.4, 2.2Hz, ArH para to cyclopentyloxy), 6.98 (1H, d, J 2.2Hz, ArH ortho to cyclopentyloxy), 7.07 (1H, d, J 5.2Hz, pyrimidine H₅), 7.15-7.45 (5H, m, C₆H₅), 8.53 (1H, d, J 5.2Hz, pyrimidine H₆), and 8.99 (1H, s, pyrimidine H₂).
d) **(±)-3-[2-(3-Cyclopentyloxy-4-methoxyphenyl)2-hydroxy-2-phenylethyl]pyridazine**
   From 3-methylpyridazine (1.0ml) and Intermediate 2 (3.98g). Purification by chromatography (SiO₂; EtOH-CH₂Cl₂) afforded the title compound (4.02g) as an off-white solid.

### INTERMEDIATE 11

### 3,5-Dichloro-4-methylpyridine

3,5-Dichloropyridine (2.04g, 13.5mmol) in THF (5ml) was added dropwise to a solution of LDA [prepared from diisopropylamine (1.9ml, 13.5mmol) and n-BuLi (1.6M; 8.4ml, 13.5mmol)] in THF (25ml) at -70°C. After stirring at this temperature for 5 min, iodomethane (0.85ml, 13.5 mmol) was added and the reaction mixture stirred for a further 1.5h at -70°C. Saturated NaHCO₃ (20ml) and CH₂Cl₂ (20ml) were added, the organic phase separated, dried (MgSO₄), and concentrated *in vacuo.* The residue was subjected to chromatography (SiO₂; Et₂O/hexane, 1:3) to afford the title compound (1,16g) as a pale yellow solid. δ_{H} (CDCl₃) 2.46 (3H, s, Me), and 8.36 (2H, s, pyridine H₂, H₆)

### INTERMEDIATE 12

### (4-Bromophenyl)(3-cyclopentyloxy-4-methoxyphenyl)ketone

A solution of Intermediate 4 (8.00g, 29.5mmol) in THF (50ml) at -70°C was treated with n-BuLi (19.4ml, 31.0mmol, 1.6M solution in hexanes). The slightly yellow solution was stirred at -70°C for 0.5h then a solution of 4-bromobenzaldehyde (5.46g, 29.5mmol) in THF (50ml) was added *via* cannula. The reaction was allowed to warm to RT over 2h then quenched with water (25ml) and extracted with Et₂O (2x50ml). The extract was dried (MgSO₄) and concentrated *in vacuo* to give a pale yellow oil which was dissolved in CH₂Cl₂ (150ml) and treated with manganese dioxide (19.24g, 0.22mol). The mixture was stirred vigorously for 20h at RT then filtered through Celite® and the residue washed with CH₂Cl₂ (5x50ml). The filtrate was concentrated *in vacuo* to give an off-white solid which was triturated with hexane to give the title compound (7.50g) as a white solid; δ_{H} (CDCl₃) 1.55-2.05 (8H, m, (CH₂)₄), 3.92 (3H, s, OMe), 4.83 (1H, m, OCH), 6.89 (1H, d, J 8.4Hz, ArH ortho to OMe), 7.33 (1H, dd, J 8.4, 2.0Hz, ArH para to OMe), 7.42 (1H, d, J 2.0Hz, ArH ortho to cyclopentyloxy), and 7.55-7.7 (4H, m, C₆H₄); ν_{max.} (CDCl₃) 2248, 1652, 1590, and 1270 cm⁻¹ ; m/z (ESI) 399 (M⁺ +2+Na, 100%), 397 (M⁺+Na, 90), 296 (16), and 236 (10).

### INTERMEDIATE 13

### Ethyl (E)-(3-Cyclopentyloxy-4-methoxyphenyl)-2-(4-pyridyl) propenoate

A mixture of Intermediate 1 (26.62g, 0.12mol), ethyl-4-pyridylacetate (19.92g, 0.12mol, 1eq) and ammonium acetate (18.63g, 0.24g, 2eq) in glacial acetic acid (200ml) was stirred at 120°C under nitrogen for 20h. The solution was cooled to RT and the acid removed *in vacuo.* The orangy/brown residue was taken up in saturated NaHCO₃ solution to pH 8.5 and the aqueous layer extracted several times with EtOAc. The combined organic layer was washed (brine), dried (MgSO₄) and evaporated to dryness to give a yellow solid. Recrystallisation from toluene/hexane (1st crop) then toluene (2nd crop) followed by column chromatography (hexane-EtOAc/hexane, 7:3) gave the title compound as a white crystalline solid. m.p. 109-110°C. δ_{H} (CDCl₃) 1.27(3H, t, J 7.1Hz, CH₂CH₃), 1.45-1.8 (8H, br m, cyclopentyl H's), 3.81 (3H, s, OMe), 4.16 (1H, br m, OCH), 4.25 (2H,q, J 7.1 Hz, CH₂CH₃), 6.43 (1H, d J 2.0 Hz, ArH ortho to cyclopentyloxy), 6.73 (1H, d, J 8.4 Hz, ArH ortho to OMe), 6.80 (1H, dd, J 2.0, 8.4 Hz, ArH para to cyclopentyloxy), 7.22 (2H, dd, J 1.6, 4.5Hz, pyridine H₃, H₅), 7.83 (1H, s, HC = C) and 8.64 (2H, dd, J 1.6, 4.5 Hz, pyridine H₂ H₆).

### INTERMEDIATE 14

### 4-(4,4-dimethyl-2-oxazolinyl)-3'-cyclopentyloxy-4'-methoxyphenyl)ketone

A solution of 2-(4-bromophenyl)-4,4-dimethyloxazoline (A. J. Meyers, D. L. Temple, D. Haidukewych and E. D. Milhelich J. Org. Chem, 39, 2787, 1974) (53.25g, 0.21mol) in THF (200ml) was added dropwise to magnesium turnings (6.0g, 0.25g atoms). The reaction was stirred for 2h at RT, then a solution of Intermediate 1 (46.0g, 0.21mol) in THF (200ml) was added dropwise. The reaction was stirred for 16h then heated to reflux for 1 h, cooled to RT and quenched with ammonium chloride solution (200ml). The layers were separated and the aqueous layer extracted with EtOAc (2x250ml). The organic layer was washed with brine (250ml), dried (MgSO₄), then concentrated *in vacuo* to give an orange oil. The crude oil was dissolved in CH₂Cl₂ (350ml) and treated with manganese dioxide (1379, 1.58mol) then stirred vigorously for 72h. The mixture was filtered through Celite® and the residue washed with CH₂Cl₂ (300ml). The filtrate was concentrated *in vacuo* and the residue triturated with Et₂O to give the title compound (59.4g) as an off white amorphous powder m.p. 159°C. δ_{H} (CDCl₃) 1.41 (6H, s, CMe₂), 1.5-2.1 (8H, m, (CH₂)₄), 3.92 (3H, s, OMe), 4.15 (2H, s, oxazoline CH₂), 4.84 (1H, m, OCH) ,6.89 (1H, d, J 8.4HZ, ArH ortho to OMe), 7.35 (1H, dd, J 2.0, 8.4 Hz, ArH para to OMe), 7.43 (1H, d, J 2 Hz, ArH ortho to cyclopentyloxy), 7.78 (2H, d, J 8.5Hz, ArH), and 8.03 (2H, d, J 8.5Hz, ArH); νₘₐₓ (CDCl₃)1648 and 1271 cm⁻¹ ; m/z (ESI) 394 (M⁺ + 1, 100%).

The following Intermediate was prepared in a manner similar to the compound of Example 7a.

### INTERMEDIATE 15

### (E) and (Z) isomers of 4-{2-(3-Cyclopentyloxy-4-methoxyphenyl)-2-[4-(4,4-dimethyl-2-oxazolinyl)phenyl]ethenyl}pyridine

From the alcohol of Intermediate 7e (4.57g, 9.8mmol), trifluoroacetic anhydride (2.47g, 1.66ml, 11.8mmol) and triethylamine (0.99g, 1.36ml, 11.8mmol). A portion (100mg) of the residue was subjected to chromatography (SiO₂; EtOAc) to give the title compound (68mg) as a yellow foam. δ_{H} (CDCl₃) 1.39, 1.41 (6H, s, CMe₂), 1.5-1.95 (8H, m, (CH₂)₄), 3.85, 3.88 (3H, s, OMe), 4.11, 4.14 (2H, s, oxazoline CH₂), 4.55, 4.69 (1H, m, OCH), 6.6-6.7 (1H, m, ArH), 6.8-6.85 (3H, m, ArH), 6.91 (1H, d, J 6.2Hz, pyridine H₃, H₅), 7.23, 7.38 (2H, d, J 8.2Hz, ArH), 7.9-8.0 (2H, m, ArH) and 8.3-8.45 (2H, m, pyridine H₂, H₆); νₘₐₓ (CDCl₃)1735, 1646, 1597 and 1318 cm⁻¹; m/z (ESI) 469 (M⁺,100%).

### EXAMPLE 1

a) **(E) and (Z) isomers of 4-[2-(3-Cyclopentyloxy-4(methoxyphenyl)-2-phenylethenyl] pyridine**
   Intermediate 7a (3.13g, 8.05mmol) was dissolved in toluene (70ml) containing 4-toluenesulphonic acid monohydrate (1.91g, 10.05mmol) and the mixture heated to reflux for 1h. The reaction mixture was poured into aqueous NaOH (10%; 100ml) and stirred for 5 min. The mixture was extracted with Et₂O (3x70ml) and the organic extract washed with water (80ml), and brine (80ml), then dried (MgSO₄), and concentrated *in vacuo* to afford a mixture of the title compounds (3.09) as a viscous pale yellow oil. δ_{H} (CDCl₃) 1.5-2.1 (8H, br m, (CH₂)₄), 3.82 (major) and 3.84 (minor) (3H, s, OMe), 4.8 (1H, br m, OCHCH₂), 6.6-7.4 (11H, m, ArH ortho to OMe + 2xArH meta to OMe + C₆H₅+ pyridine H₃, H₅), and 8.2 - 8.35 (2H, m, pyridine H₂, H₆); m/z 372 (M⁺ + 1, 12%), 371 (M⁺, 40), 304 (21), 303 (100), 302 (72) and 274 (22).
   The following compounds were prepared using a similar procedure:
b) **(E) and (Z) isomers of 2-[2-(3-Cyclopentyloxy-4-methoxyphenyl]-2-phenylethenyl]pyrazine**
   From Intermediate 7b (570mg, 1.5mmol) and 4-toluene sulphonic acid (about 20mg). Upon completion, the reaction mixture was concentrated *in vacuo* then subjected to chromatography (SiO₂; Et₂O) to afford the title compound (520mg) as a colourless oil. δ_{H} (CDCl₃) 1.5-2.0 (8H, br m, (CH₂)₄), 3.84 and 3.86 (3H, s, OMe), 4.58 and 4.72 (1H, br m, OCH), 6.65-7.5 (9H, m, C₆H₅+C=CH+ArH ortho to OMe+2xArH meta to OMe), 7.90 and 8.04 (1H, d, J 1.5Hz, pyrazine H₃), 8.18 and 8.21 (1H, d, J 2.5Hz, pyrazine H₆), and 8.45 and 8.48 (1H, m, pyrazine H₅).
c) **(E) and (Z) isomers of 3-[2-(3-Cyclopentyloxy-4-methoxyphenyl)-2-phenylethenyl] -2-methoxypyrazine**
   From Intermediate 10a (2.94g, 7.0mmol) and 4-toluene-sulphonic acid (about 20mg) as described for Intermediate 7b to afford the title compound (2.67g) as a yellow oil. δ_{H} (CDCl₃) 1.5-2.0 (8H, br m, (CH₂)₄), 3.80, 3.81, 3.83, 3.86 (2 x 3H, s, 2 x OMe), 4.50, 4.70 (1H, br m, OCH), 6.60-7.5 (9H, m, C₆H₅ + C = CH + ArH ortho to OMe + 2 x ArH meta to OMe) and 7.7-7.95 (2H, m, pyrazine H₅, H₆).
d) **(i) (E) 4-[2-(3-Cyclopentyloxy-4-methoxyphenyl)-2-phenylethenyl]** **3,5-dichloropyridine**
   **(ii) (Z) 4-[2-(3-Cyclopentyloxy-4-methoxyphenyl)-2-phenylethenyl] -3,5-dichloropyridine**
   From Intermediate 7c (1.60g, 3.58mmol) and 4-toluene-sulphonic acid (0.85g). Purification by column chromatography (SiO₂; CH₂Cl₂) afforded: i) (E) title compound (960mg) as an off-white solid m.p. 138.5-140°C, δ_{H} (CDCl₃) 1.5-2.0 (8H, br m, (CH₂)₄), 3.88 (3H, s, OMe), 4.72 (1H, br m, OCH), 6.59 (1H, s, C=CH), 6.85 (1H, d, J 8.4Hz, ArH ortho to OMe), 6.90 (1H, d, J 2.0Hz, ArH ortho to cyclopentyloxy), 6.95 (1H, dd, J 8.4, 2.0Hz, ArH para to cyclopentyloxy), 7.0-7.1 (2H, m, H₂, H₆ of C₆H₅), 7.15-7.3 (3H, m, H₃, H₄, H₅ of C₆H₅), and 8.35 (2H, s, pyridine H₂, H₆). and ii) (Z) title compound (240mg) as an off-white solid. m.p. 155-156.5°C. δ_{H} (CDCl₃) 1.4-1.8 (8H, br m (CH₂)₄), 3.80 (3H, s, OMe), 4.42 (1H, br m OCH), 6.52 (1H, d, J 2.0 OHz,m ArH ortho to cyclopentyloxy), 6.56 (1H, s, C=CH), 6.57 (1H, dd, J 8.4, 2.0 Hz, ArH para to cyclopentyloxy), 6.68 (1H, d, J 8.4 Hz, ArH ortho to OMe), 7.3-7.45 (5H,m, C₆H₅), and 8.37 (2H, s, pyridine H₂, H₆).
e) **(E) and (Z) isomers of 2-[2-(3-Cyclopentyloxy-4-methoxy phenyl))-2-phenylethenyl]-4-methylpyridine**
   From Intermediate 10b (1.15g, 2.85mmol). Purification by chromatography (SiO₂; EtOAc) afforded the title compound (1.29) as a pale yellow solid; δ_{H} (CDCl₃) 1.4-1.9 (8H, br m, (CH₂)₄), 2.04 (major), 2.09 (minor) (3H, s, pyridine Me), 3.85 (major), 3.88 (minor) (3H, s, OMe), 4.58 (minor), 4.72 (major) (1H, br m, OCH), 6.4-7.5 (11H, m, C₆H₅+C₆H₃+pyridine H₃, H₅ + C=CH), 8.5-8.55 (1H, m, pyridine H₆). 'Hn.m.r indicates a 2:1 E/Z ratio.
f) **(E) and (Z) isomers of 4-[2-(3-Cyclopentyloxy-4-methoxy phenyl)-2-phenylethenyl]pyrimidine**
   From Intermediate 10c (2.55g). Purification by chromatography (SiO₂; Et₂O) afforded the title compound (1.20g) as a pale yellow foam; δ_{H} (CDCl₃) 1.5-2.0 (8H, br m, (CH₂)₄), 3.88,3.90 (3H, s, OMe), 4.60, 4.70 (1H, br m, OCH), 6.44, 6.64 (1H, d, J 5.2Hz, pyrimidine H₅), 6.65-7.0 (3H, m, C₆H₃), 7.2-7.45 (6H, m, C₆H₅+C=CH ), 8.26, 8.32 (1H, d, J 5.2Hz, pyrimidine H₆) , and 9.10, 9.12 (1H, ca s, pyrimidine H₂).
g) **(E) and(Z) isomers of 3-[2-(3-Cyclopentyloxy-4-methoxy phenyl)-2-phenylethenyl]pyridazine**
   From Intermediate 10d (4.0g). Purification by chromatography (SiO₂; Et₂O) afforded the title compound (2.07g) as a pale yellow solid (Found: C, 77.59; H, 6.49; N, 7.24. C₂₄H₂₄N₂O₂ requires C, 77.39; H, 6.50; N, 7.52%); δ_{H} (CDCl₃) 1.5-1.9 (8H, br m, (CH₂)₄), 3.88 ,3.90 (3H, s, OMe), 4.58, 4.70 (1H, br m, OCH), 6.6-7.5 (11H, m, C₆H₅+C₆H₃+C=CH + pyridazine H₄, H₅+ ), and 8.85-8.90 (1H, m, pyridazine H₆) ('Hnmr indicates a 3:2 E/Z ratio); m/z (ESI) 396 (M⁺+1+Na, 57%), 395 (M⁺+Na,100), 374 (66), 373 (78), and 305 (16).

### EXAMPLE 2

### (E) and (Z) isomers of 4-[1-(3-Cyclopentyloxy-4-methoxyphenyl)-2-(4-pyridyl)ethenyl] pyridine

A mixture of Intermediate 6 (0.48g,1.58mmol), Cs₂CO₃ (0.56g,1.73mmol), and cyclopentyl bromide (0.26g, 1.743mmol) in DMF (20ml) was stirred at RT overnight. A further portion of Cs₂CO₃ (0.20g,0.61 mmol) and cyclopentyl bromide (0.28g, 1.86mmol) was added, the mixture stirred for 1.5h then concentrated *in vacuo.* The residue was subjected to chromatography (SiO₂; EtOAC/CH₃OH/Et₃N, 100:1:0.4) to afford the title compound (0.42g) as a white solid. m.p. 136-138°C (cyclohexane); δ_{H} (CDCl₃) 1.5-2.0 (8H, br m (CH₂)₄), 3.84 (3H, s, OMe), 4.65 (1H, br m OCHCH₂), 6.7-6.9 (6H, m, ArH ortho to OMe+2xArH meta to OMe+C=CH + pyridine H₃,H₅), 7.08 (2H, dd, J 4.5, 1.5 Hz, pyridine pyridine H₃, H₅'), 8.32 (2H, dm, J 5.0 Hz pyridine H₂, H₆), and 8.55 (2H, dd, J 4.5, 1.5Hz, pyridine H₂, H₆^{,}); m/z 372 (M⁺28%), 305 (37), 304 (100), 303 (95), 275 (18), and 41 (18)

### EXAMPLE 3

a) **(E) and (Z) isomers of 4-[2-(3-Cyclopentyloxy-4-methoxy phenyl)-2-phenylethenyl] phenol**
   A mixture of Intermediate 9 (2.94g, 10mmol), 4-bromophenol (2.16g, 12.5mmol), Et3N (2.52g, 25mmol), tri-o-tolyl phosphine (0.06g, 0.2mmol) and palladium acetate (0.022g, 0.1mmol) was heated in a bomb at 140°C for 16h. Upon cooling, the reaction mixture was diluted with NH₄Cl (10%; 50ml) and CH₂Cl₂ (50ml). The organic layer was separated and the aqueous layer extracted with CH₂Cl₂ (50ml). The combined organic layer was dried (MgSO₄), filtered and concentrated. Purification by column chromatography (SiO₂; hexane/Et₂O,1:1) yielded the title compound (1:1 mixture of isomers) (0.8g) as a yellow foam. δ_{H} (CDCl₃) 1.2-1.9 (8H, br m, (CH₂)₄), 3.81, 3.83 (3H, s, OMe), 4.59, 4.69 (1H, br m, OCH), 5.5, 5.63 (1H, br s, OH), 6.55-7.0 (8H, m, C₆H₃+C₆H₄+C=CH), and 7.15-7.35 (5H, m, C₆H₅) [N.B. 'Hn.m.r. indicates ca 1:1E/Z mixture of isomers); m/z (ESI) 410 (M⁺+1+Na,18%), 409 (M⁺+Na,100) 387 (M⁺+1, 62), 319 (38), 318 (22), 301 (19), 236 (22), and 135 (20).
   The following compounds were prepared using a similar procedure:
b) **(E) and (Z) isomers of 3-[2-(3-Cyclopentyloxy-4-methoxy phenyl)-2-phenylethenyl] benzoic acid**
   From Intermediate 9 (2.94g, 10mmol) and 3-bromobenzoic acid (5.03g, 25mmol). Purification by column chromatography [SiO₂;10%, CH₃OH/ CH₂Cl₂] furnished the title compounds (2g) as a viscous yellow oil. δ_{H} (CDCl₃) 1.45-2.0 (8H, br m, (CH₂)₄), 3.86, 3.87 (3H, s, OMe), 4.55, 4.7 (1H, br m, OCH), 6.65-8.25 (13H, m, C₆H₅+C₆H₄+C₆H₃+C=CH), (CO₂H not observed) [N.B. 'Hn.m.r. indicates ca 1:1E/Z mixture of isomers]; m/z (ESI) 437 (M⁺+23, 60%), 301 (67), 281 (100), and 259 (52).
c) **(E) and (Z) isomers of 4-[2-(3-Cyclopentyloxy-4-methoxy phenyl)-2-phenylethenyl] anisole**
   From Intermediate 9 (1.19g, 4.04mmol) and 4-bromoanisole (0.757g, 4.05mmol). Purification by column chromatography [SiO₂; hexane/Et₂O, 4:1] furnished the title compounds (0.78g) as a yellow oil. δ_{H} (CDCl₃) 1.5-2.0 (8H, br m, (CH₂)₄), 3.72, 3.73 (3H, s, OMe), 3.82, 3.86 (3H, s, OMe), 4.58, 4.67 (1H, br m, OCH), 6.6-6.9 (6H, m, C₆H₃+2xArH ortho to OMe+C=CH), 6.93, 7.00 (2H, d, J 8.5Hz, 2xArH meta to OMe) and 7.15-7.35 (5H, m, C₆H₅) [N.B. 'Hn.m.r. indicates ca 1:1E/Z mixture of isomers]; m/z (ESI) 424 (M⁺+1+Na, 20%), 423 (M⁺+Na, 100%), 374 (12), 281 (20), 198 (12), 132 (12) and 86 (12).
d) **(E) and (Z) isomers of Methyl 4-[2-(3-Cyclopentyloxy-4** **methoxyphenyl)-2-phenylethenyl]benzoate**
   From Intermediate 9 (2.94g, 10mmol) and methyl 4-bromobenzoate (2.69g, 12.5mmol) to afford the title compounds (3.35g) as a yellow gum; δ_{H} (CDCl₃) 1.4-2.0 (8H, br m, (CH₂)₄), 3.86, 3.87 (6H, s, OMe+CO₂Me), 4.54, 4.67 (1H, br m, OCH), 6.6-7.4 (11H, m, C₆H₅+C₆H₃+C=CH+2xArH meta to CO₂Me), and 7.75-7.85 (2H, m, 2xArH ortho to CO₂Me) [N.B. 'Hn.m.r. indicates ca 1:1 E/Z mixture of isomers]; m/z (ESI) 429 (M⁺+1+Na, 28%), 362 (18), 361 (28), 330 (70), and 329 (68).
e) **(E) and (Z) isomers of 3-[2-(3-Cyclopentyloxy-4-methoxyphenyl)-2-phenylethenyl]pyridine**
   From Intermediate 9 (1.00g, 3.4mmol) and 3-bromopyridine (1.28g, 8.1 mmol). Purification by chromatorgaphy (SiO₂; Et₂O) afforded the title compound (0.50g) as a pale yellow gum; δ_{H} (CDCI₃) 1.45-2.0 (8H, br m, (CH₂)₄), 3.85 (major), 3.87 (minor) (3H, s, OMe), 4.55 (minor), 4.69 (major) (1H, br m, OCH), 6.65-7.5 (11H, m, C₆H₅+C₆H₃+pyridine H₄,H₅+ C=C), and 8.2-8.45 (2H, m, pyridine H₂,H₆).

### EXAMPLE 4

### (E) and (Z) isomers of 4-[2-(3-Cyclopentyloxy-4-methoxyphenyl)-2-phenylethenyl] acetoxybenzene

To a stirred solution of the compound of Example 3a (0.2g, 0.52mmol) in CH₂Cl₂ (5ml), under a nitrogen atmosphere, was added Et₃N (0.101g, 0.14ml, 1mmol) followed by acetyl chloride (0.0785g, 0.071ml, 1mmol). The reaction mixture was stirred at RT for 4h then poured into saturated NaHCO₃ (10ml). The organic layer was separated and the aqueous layer extracted with CH₂Cl₂. The combined organic layer was dried (MgSO₄), filtered, and the solvent removed *in vacuo* to furnish the title compounds (0.222g) as a colourless oil. δ_{H} (CDCl₃) 1.5-1.9 (8H, br m, (CH₂)₄), 2.23, 2.24 (3H, s, OCOMe), 3.83, 3.86 (3H, s, OMe), 4.56, 4.67 (1H, br m, OCH), and 6.7-7.4 (13H, m, C₆H₅+C₆H₄+C₆H₃+C=CH) [N.B. 'Hn.m.r. indicates ca 1:1E/Z mixture of isomers]; m/z (ESI) (M⁺+Na, 100% ), 319 (20), 281 (29), 191 (48), 127 (50) and 55 (54).

### EXAMPLE 5

### (E) and (Z) isomers of Methyl 3-[2-(3-cyclopentyloxy-4-methoxy

### phenyl)-2-phenylethenyl]benzoate

To a cold (0°C) solution of the compound of Example 3b (0.25g, 0.6mmol) in CH₃OH (20ml) was added SOCl₂ (0.357g,0.22ml,3mmol) dropwise and the reaction mixture was stirred at RT for 3h. The solvent was evaporated *in vacuo,* the residue dissolved in CH₂Cl₂ (20ml) and washed with saturated NaHCO₃ (20ml). The organic phase was separated and the aqueous phase extracted with CH₂Cl₂ (20ml). The combined organic layer was dried (MgSO₄), filtered and the solvent evaporated *in vacuo* to yield the title compound (0.215g) as a yellow oil. δ_{H} (CDCl₃) 1.4-2.0 (8H, br m, (CH₂)₄), 3.82, 3.83, 3.84, 3.85 (6H, s, OMe + CO₂Me), 4.54, 4.69 (1H, br m, OCH), and 6.65-7.85 (13H, m, C₆H₅+C₆H₄+C₆H₃+C=CH) [N.B. 'Hn.m.r. indicates ca 1:1E/Z mixture of isomers]; m/z (ESI) 429 (M⁺+1, 25%), 361 (22), 329 (100), 159 (12), 102 (15), and 60 (75).

### EXAMPLE 6

### (E) and (Z) isomers of 4-[2-(4-Aminophenyl)-2-(3-cyclopentyloxy-4-methoxyphenyl)ethenyl]pyridine

Water (15ml) and trifluoroacetic acid (10ml) were added to Intermediate 13 (6.1g) in CH₂Cl₂ (15ml) at O°C and the mixture allowed to warm to RT. After 6h, the reaction mixture was concentrated *in vacuo* and the residue partitioned between 10% hydrochloric acid (50ml) and EtOAc (50ml). The aqueous layer was separated, basified to pH 14 with 20% sodium hydroxide solution, and extracted with CH₂Cl₂ (3x50ml). The extract was dried (MgSO₄) and concentrated *in vacuo* to give the crude title compound (4.2g). A portion (0.40g) was subjected to chromatography (SiO₂); EtOAc) to afford the title compound (0.29g); δ_{H} (CDCl₃) 1.45-2.0 (8H, br m, (CH₂)₄), 3.80 (2H, br s, NH₂), 3.87, 3.90 (3H, s, OMe), 4.58, 4.70 (1H, br m, OCH), 6.6-7.2 (10H, C₆H₄+ C₆H₃+pyridine H₃, H₅+C=CH), and 8.3-8.4 (2H, m, pyridine H₂,H₆); m/z (ESI) 388 (M⁺+1, 100%).

### EXAMPLE 7

a) **(E) and (Z) isomers of 4-[2-(4-Bromophenyl)-2-(3-cyclopentyloxy-4-methoxyphenyl)ethenyl]pyridine**
   A solution of Intermediate 7d (7.52g, 16.0mmol) and triethylamine (4.05g, 5.60ml, 40.0mmol) in CH₂Cl₂ (100ml) was cooled to 0°C and trifluoroacetic anhydride (3.70g, 2.50ml, 17.6mmol) was added dropwise. The orange-red solution was allowed to warm to RT over 20h then water (25ml) was added. The mixture was extracted with CH₂Cl₂ and the extract was dried (MgSO₄), concentrated *in vacuo* and subjected to chromatography to give the title compound (4.73g) as a white amorphous powder. (Found: C, 66,66; H, 5.27; N, 2.99. C₂₅H₂₄BrNO₂ requires C, 66.67; H, 5.37; N, 3.11%); &_{H}(CDCl₃) 1.45-1.95 (8H, br, m, (CH₂)₄), 3.86, 3.88 (3H, s; OMe), 4.55, 4.70 (1H, br m, OCH), 6.6-6.95 (6H, m, C₆H₃, + pyridine H₃, H₅) + C=CH), 7.06, 7.21 (2H, d, J 8.4Hz,ArH of C₆H₄), 7.4-7.5 (2H,m,ArH of C₆H₄), and 8.36 (2H, ca. d, J 6.0Hz, pyridine H₂, H₆) ('H n.m.r. indicates a 1:1 E/Z mixture); νₘₘ (CDCl₃) 1597, 1514, and 1251 cm⁻¹; m/z (ESI) 452 (M⁺ + 2 + Na, 100%), 450 (M⁺ +Na, 88), 384 (30) and 382 (28):

### EXAMPLE 8

### (E) and (Z) isomers of -4-[-1-(3-Cyclopentyloxy-4-methoxyphenyl)-2-(4-pyridyl)ethenyl]benzoic acid hydrochloride

Intermediate 15 (4.25gm 8.8mmol) in 10% aqueous HCI (15ml) was heated to reflux for 20 min. Aqueous NaOH solution (5M; 20ml) and EtOH (15 ml) were then added and heating continued for a further 2h. The reaction was cooled to RT and acidified to pH 1 with 10% aqueous HCI. The mixture was extracted with CHCI₃ (10x100ml), the organic extract was dried (MgSO₄) and concentrated *in vacuo* to give the title compound (2.83g) as a yellow solid; δ_{H} (d₄-MeOH) 1.45, 1.8 (8H, br m, (CH₂)₄), 3.86, 3.88 (3H, s, OMe), 4.66, 4.74 (1H, br m, OCH). 6.65-7.65 (8H, m, C=CH + C₆H₃ + pyridine H₃, H₅, + ArH meta to CO₂H), 8.05, 8.13 (2H, d, J ca .8Hz, ArH ortho to CO₂H), and 8.46, 8.55 (2H, d, J ca. 6Hz, pyridine H₂, H₆) (N.B. CO₂H and HCl not observed) (2H, d, J 8.2Hz, ArH); νₘₐₓ (Nujol) 1710, and 1633 cm⁻¹ ; m/z (ESI) 416 (M⁺ ₊₁, 100%).

### EXAMPLE 9

### (E) and (Z) isomers of t-Butyl N-{4-[1-(3-cyclopentyloxy-4-methoxyphenyl)-2-(4-pyridyl)ethenyl]phenyl}carbamate

A mixture of diphenyl phosphoryl azide (0.61g, 0.48ml, 2.2mmol), Intermediate 14 (1.00g, 2.2mmol), triethylamine (0.49, 0.68ml, 4.9mmol) and t-butanol (25ml) was heated to reflux for 20h. The mixture was concentrated *in vacuo* and the resulting brown oil partitioned between CH₂Cl₂ (30ml) and 5% citric acid solution (30ml). The organic layer was separated, washed with water (20ml), NaHCO₃ solution (20ml), and brine (20ml), then dried (MgSO₄) and concentrated *in vacuo* to give a red oil; which was subjected to chromatography to give the title compound (0.60g, 56%) as a yellow foamy solid. δ_{H} (CDCl₃) 1.52, 1.54 (9H, s, CMe₃), 1.65-1.9 (8H, br m, (CH₂)₄), 3.86, 3.89 (3H, s, OMe), 4.56, 4.70 (1H, m, OCH), 6.6-7.4 (11H, m, ArH + C=CH+NCO), and 8.34 (2H, d, J 5.2Hz, pyridine H₂, H₆). ('H n.m.r. indicates ca. 1:1 mixture of isomers); νₘₐₓ (CHCl₃) 3441, 1730, 1596, 1518 and 1157 cm⁻¹ ; m/z (ESI) 487 (M⁺ +1, 75%), 472 (12), and 431 (100).

### EXAMPLE 10

a**) (E) and (Z) isomers of 2- Cyclopentyloxy-4-[2-(4-fluorophenyl)-1-phenylethenyl]anisole**
   Dimethyl 4-fluorobenzylphosphonate (432mg, 2.0ml) was aded to a mixture of sodium hydride (80mg, 2.2mmol) and Intermediate 2 (592mg, 2.0mmol) in THF (5ml) at 0°C. The reaction mixture was stirred ovemight at RT then quenched with NaHCO₃ solution (5ml) and extracted with Et₂O (25ml). The extract was washed with brine (10ml), dried (MgSO₄), and concentrated *in vacuo* to afford the title compound (115mg) as a colourless gum; δ_{H} (CDCl₃) 1.5-1.9 (8H, br m, (CH₂)₄), 3.84, 3.86 (3H, s, OMe), 4.56,4.67 (1H, br m, OCH), and 6.65-7.4 (13H, m, C₆H₃ + C₆H₄ + C₆H₅ + C=CH); m/z (ESI) 390 (M⁺ + 2, 23%), 389 (M⁺+1, 92), 253 (37), and 235 (100).
   The following compound was prepared in a manner similar to the compound of Example 10a.
b) **(E) and (Z) isomers of 4-[2-(4-chlorophenyl)-1-phenylethenyl]-2-cyclopentyloxyanisole**
   From Intermediate 2 and diethyl 4-chlorobenzylphosphonate to afford the title compound as a clear oil. δ_{H} (CDCl₃) 1.5-2.0 (8H, br m, (CH₂)₄), 3.86, 3.89 (3H, s, OMe), 4.57,4.70 (1H, br m, OCH), and 6.65-7.4 (13H, m, C₆H₃ + C₆H₄ + C₆H₅ + C=CH) ('Hn.m.r. indicates ca 1:1 E:Z ratio); m/z (ESI) 429 (M⁺ + 2 + Na, 15%), 427 (M⁺+Na, 45), 387 (30), 386 (100), 301 (25), and 60 (20).

### FORMULATION EXAMPLES

The compounds of the invention may be formulated for pharmaceutical use in a number of forms using any suitable excipients. Thus, for example, for oral use the compounds of the invention such as the compounds of the Examples may be formulated as a solid dosage form, by mixing an appropriate weight of compound (for example 50mg) with maize starch (50-99%w/w), anhydrous colloidal silica (0-10%w/w) and organic or inorganic acid (up to 1%w/w), to fill capsules of an appropriate size, e.g. white opaque hard gelatine capsules size 3. If desired the same mixture may be compressed into tablets.

The activity and selectivity of compounds according to the invention was demonstrated in the following tests. In these tests the abbreviation FMLP represents the peptide N-formyl-met-leu-phe.

### Isolated Enzyme

The potency and selectivity of the compounds of the invention was determined using distinct PDE isoenzymes as follows:
i. PDE I, rabbit heart
ii. PDE II, rabbit heart
iii. PDE III, rabbit heart, Jurkat cells
iv. PDE IV, HL60 cells, rabbit brain, rabbit kidney and human recombinant PDE IV
v. PDE V, rabbit lung, guinea pig lung

A gene encoding human PDE IV has been cloned from human monocytes (*Livi, et al., 1990, Molecular and Cellular Biology, 10, 2678).* Using similar procedures we have cloned human PDE IV genes from a number of sources including eosinophils, neutrophils, lymphocytes, monocytes, brain and neuronal tissues. These genes have been transfected into yeast using an inducible vector and various recombinant proteins have been expressed which have the biochemical characteristics of PDE IV (*Beavo and Reifsnyder, 1990, TIPS, 11 150*). These recombinant *enzymes,* particularly the human eosinophil recombinant PDE IV, have been used as the basis of a screen for potent, selective PDE IV inhibitors.

The enzymes were purified to isoenzyme homogeneity using standard chromatographic techniques.

Phosphodiesterase activity was assayed as follows. The reaction was conducted in 150µl of standard mixture containing (final concentrations): 50mM 2-[[tris(hydroxymethyl)methyl]amino]-1-ethane-sulphonic acid (TES) -NaOH buffer (pH 7.5), 10mM MgCl₂, 0.1µM [³H]-cAMP and vehicle or various concentrations of the test compounds. The reaction was initiated by addition of enzyme and conducted at 30°C for between 5 to 30 mins. The reaction was terminated by addition of 50µl 2% trifluoroacetic acid containing [¹⁴C]-5'AMP for determining recovery of the product. An aliquot of the sample was then applied to a column of neutral alumina and the [³H]-cAMP eluted with 10ml 0.1 TES-NaOH buffer (pH8). The [³H]-5'-AMP product was eluted with 2ml 2M NaOH into a scintillation vial containing 10ml of scintillation cocktail. Recovery of [³H]-5'AMP was determined using the [¹⁴C]-5'AMP and all assays were conducted in the linear range of the reaction.

Compounds according to the invention such as compounds of the Examples herein cause a concentration-dependent inhibition of recombinant PDE IV at 0.1 - 1000nM with little or no activity against PDE I, II, III or V at concentrations up to 100µM.

### 2. The Elevation of cAMP in Leukocytes

The effect of compounds of the invention on intracellular cAMP was investigated using human neutrophils or guinea pig eosinophils. Human neutrophils were separated from peripheral blood, incubated with dihydrocytochalasin B and the test compound for 10 min and then stimulated with FMLP. Guinea pig eosinophils were harvested by peritoneal lavage of animals previously treated with intraperitoneal injections of human serum. Eosinophils were separated from the peritoneal exudate and incubated with isoprenaline and test compound. With both cell types, suspensions were centrifuged at the end of the incubation, the cell pellets were resuspended in buffer and boiled for 10 min prior to measurement of cAMP by specific radioimmunoassay (DuPont).

The most potent compounds according to the Examples induced a concentration -dependent elevation of cAMP in neutrophils and/or eosinophils at concentrations of 0.1nM to 1µM.

### 3. Suppression of Leukocyte Function

Compounds of the invention were investigated for their effects on superoxide generation, chemotaxis and adhesion of neutrophils and eosinophils. Isolated leukocytes were incubated with dihydrocytochalasin B for superoxide generation only and test compound prior to stimulation with FMLP. The most potent compounds of the Examples caused a concentration-dependent inhibition of superoxide generation, chemotaxis and adhesion at concentrations of 0.1nM to 1µM.

Lipopolysaccharide (LPS)-induced synthesis of tumour necrosis factor (TNF) by human peripheral blood monocytes (PBM) is inhibited by compounds of the Examples at concentrations of 0.01nM to 10µM.

### 4. Relaxation of Constricted Airway Smooth Muscle in vitro

The effects of compounds of the invention on guinea-pig isolated tracheal smooth muscle were investigated. Isolated tracheal rings were suspended in organ baths and immersed in oxygenated Krebs' solution. The smooth muscle was contracted with sub-maximal concentrations of histamine or carbachol prior to the addition of increasing concentrations of test compound to the organ baths. The most potent compounds of the Examples caused a concentration-dependent reversal of both histamine and carbachol-induced contractions at concentrations of 1nM to 100µM. The compounds were generally more potent in reversing histamine-induced tone than carbachol-induced tone.

### 5. Effects on Cardiac Muscle in vitro

Compounds of the invention have been tested for their effects on isolated cardiac muscle. Right atrial and papillary muscles were dissected out from the hearts of guinea pigs and suspended in organ baths for measuring the rate (chronotropic) of spontaneously beating atria and force (inotropic) of the electrically stimulated papillary muscle. In these preparations, selective PDE IV inhibitors such as rolipram do not have any direct effects whereas selective PDE III inhibitors such as milrinone have positive chronotropic and inotropic effects. The non-specific PDE inhibitor theophylline, which is used in asthma as a bronchodilator, also causes significant cardiovascular changes such as tachycardia. Selective PDE IV inhibitors have advantage over theophylline, therefore, through reduced cardiovascular side effects. The most potent and selective compounds of the Examples had no direct effects on the atrial and papillary muscles in vitro at concentrations up to 10µM but in combination with PDE III inhibitors, these inhibitors showed an enhancement of chronotropic and inotropic activity, typical of selective type IV inhibitors.

### 6. Anti-inflammatory Activity in vivo

Interleukin-5 (IL-5)-induced pleural eosinophilia in the rat (*Lisle, et al, 1993, Br.J. Pharmacol. 108, 230p*) is inhibited by compounds of the Examples given orally at doses of 0.0001 to 10.0mg/kg. The most potent compounds cause a dose-dependent reduction in migrating eosinophils with ED₅₀s of 0.003 to 0.03mg/kg p.o.

Compounds of the invention also reduce the inflammatory responses induced in rats by platelet activating factor (PAF).

### 7. Anti-allergic Activity in vivo

Compounds of the invention have been tested for effects on an IgE-mediated allergic pulmonary inflammation induced by inhalation of antigen by sensitised guinea pigs. Guinea pigs were initially sensitised to ovalbumin under mild cyclophosphamide-induced immunosuppression, by intraperitoneal injection of antigen in combinations with aluminium hydroxide and pertussis vaccine. Booster doses of antigen were given two and four weeks later and at six weeks, animals were challenged with aerosolised ovalbumin whilst under cover of an intraperitoneally administered anti-histamine agent (mepyramine). After a further 48h, bronchial alveolar lavages (BAL) were performed and the numbers of eosinophils and other leukocytes in the BAL fluids were counted. The lungs were also removed for histological examination for inflammatory damage. Administration of compounds of the Examaples (0.001-10mg/kg i.p. or p.o.), up to three times during the 48h following antigen challenge, lead to a significant reduction in the eosinophilia and the accumulation of other inflammatory leukocytes. There was also less inflammatory damage in the lungs of animals treated with compounds of the Examples.

### 8. Effects on Pulmonary Dynamics

Compounds of the invention (0.001-10mg/kg by oral or other route of aministration) reduce the allergic bronchoconstruction caused by antigen in sensitized guinea pigs.

Compounds of the invention have been tested for their effects on ozone-induced hyperreactivity of the airways of guinea pigs. Following the inhalation of ozone, guinea pigs become very much more sensitive to the bronchoconstrictor effects of inhaled histamine than naive animals (*Yeadon et al, 1992, Pulmonary Pharm., 5*, *39*). There is a pronounced shift to the left (10-30 fold) of the dose response curve to histamine and a highly significant increase in the maximum increase in pulmonary resistance. Compounds of the Examples administered 1h prior to ozone by the intraperitoneal or oral (0.001-10mg/kg) route caused a dose-dependent inhibition of ozone-induced hyperreactivity.

### 9. Adverse Effects

Compounds of the invention are free from adverse effects following repeated overdosage to rats or dogs. For example, over administration of 125mg/kg/day of active compounds of the Examples to rats for 30 days is not associated with adverse toxicity.

The most potent compounds of the invention are 20-30 times less active than rolipram in inducing behavioural changes, sedation or emesis in rats, ferrets or dogs.

## Claims

1. A compound of formula (1) 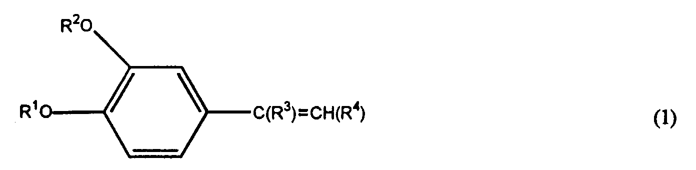
wherein R¹ is a methyl group optionally substituted by one, two or three fluorine or chlorine atoms;
R² is a C₃₋₈ cycloalkyl group optionally substituted by one, two or three substituents selected from halogen atoms, C₁₋₆ alkyl, hydroxyl or C₁₋₆ alkoxy group;
R³ and R⁴, which may be the same or different, is each a group Ar, where Ar is a phenyl, pyridyl, pyridazinyl, pyrimidinyl or pyrazinyl group, each of said groups being optionally substituted by one, two or more substituents selected from an atom or group R⁸ or Alk¹H or -Alk¹(R⁸)ₘ wherein R⁸ is a halogen atom, or an amino (-NH₂), substituted amino, nitro, cyano, hydroxyl (-OH), substituted hydroxyl, cycloalkoxy, formyl [HC(O)-], carboxyl (-CO₂H), esterified carboxyl, thiol (-SH), substituted thiol, -C(O)Alk¹H, -SO₃H, -SO₂Alk¹H, -SO₂NH₂, -SO₂NHAlk¹H, -SO₂N[Alk¹H]₂, -CONH₂, -CONHAlk¹H, -CON[Alk¹H]₂, -NHSO₂H, -NHSO₂Alk¹H, -N[SO₂Alk¹H]₂, -NHSO₂NH₂, -NHSO₂NHAlk¹H, -NHSO₂N[Alk¹H]₂, -NBC(O)Alk¹H, or -NHC(O)OAlk¹H group; Alk¹ is a straight or branched C₁₋₆alkylene, C₂₋₆alkenylene, or C₂₋₆alkynylene chain optionally interrupted by one, two or three -O- or -S-atoms or -S(O)p- [where p is an integer 1 or 2] or N(R⁶)-[where R⁶ is a hydrogen atom or C₁₋₆alkyl group] groups; and m is zero or an integer 1, 2 or 3;
said substituted amino group being a group -NH[Alk¹(R^{8a})ₘ] [where Alk¹ and m are as defined above and
R^{8a} is as defined above for R⁸ but is not a substituted amino, a substituted hydroxyl or a substituted thiol group] or a group -N[Alk¹(R^{8a})ₘ]₂ wherein each -Alk¹(R^{8a})ₘ group is the same or different;
said substituted hydroxyl or substituted thiol group being a group -OAlk¹(R^{8a})ₘ or -SAlk¹(R^{8a})ₘ respectively, where Alk¹, R^{8a} and m are as just defined; and the salts, solvates, hydrates and N-oxides thereof.

2. A compound according to claim 1 wherein R¹ is a methyl group.

3. A compound according to claim 1 or claim 2 wherein R² is a cyclopentyl group.

4. A compound according to any preceding claim wherein R³ and/or R⁴ is a substituted or unsubstituted pyridyl group.

5. A compound according to claim 4 wherein R³ and R⁴ is each a pyridyl or a monosubstituted or disubstituted pyridyl group, or R³ is a phenyl or substituted phenyl group and R⁴ is a pyridyl or a monosubstituted or disubstituted pyridyl group.

6. A compound according to claim 5 wherein said pyridyl groups are 4-pyridyl, 3-monosubstituted-4-pyridyl, 3,5-disubstituted-4-pyridyl, 2- or 4-monosubstituted-3-pyridyl or 2,4-disubstituted-3-pyridyl groups.

7. A compound according to claim 1 selected from the (E) and (Z) isomers of:
4-[2-(3-cyclopentyloxy-4-methoxyphenyl)-2-phenylethenyl]pyridine;
4-[1-(3-cyclopentyloxy-4-methoxyphenyl)-2-(4-pyridyl)ethenyl]pyridine;
4-[2-(3-cyclopentyloxy-4-methoxyphenyl)-2-(4-fluorophenylethenyl]pyridine;
4-[2-(3-cyclopentyloxy-4-methoxyphenyl)-2-(4-trifluoromethylphenyl)ethenyl]pyridine;
4-[2-(3-cyclopentyloxy-4-methoxyphenyl)-2-(2-methoxyphenylethenyl)]pyridine;
4-[2-(3-cyclopentyloxy-4-methoxyphenyl)-2-(4-methoxyphenyl)ethenyl]pyridine;
4-[2-(3-cyclopentyloxy-4-methoxyphenyl)-2-(4-methylphenyl)ethenyl]pyridine;
4-[2-(3-cyclopentyloxy-4-methoxyphenyl)-2-(3-methylphenyl)ethenyl]pyridine;
4-[2-(3-cyclopentyloxy-4-methoxyphenyl)-2-(3-cyclopentyloxy-4-methoxyphenyl)ethenyl]pyridine;
4-[2-(3-cyclopentyloxy-4-methoxyphenyl)-2-phenylethenyl] -3,5-dichloropyridine;
2-[2-(3-cyclopentyloxy-4-methoxyphenyl)-2-phenylethenyl]pyridine;
4-[1-(3-cyclopentyloxy-4-methoxyphenyl)-2-(4-pyridyl)ethenyl]aniline;
4-[1-(3-cyclopentyloxy-4-methoxyphenyl)-2-(4-pyridyl)ethenyl]benzoic acid;
ethyl N-{4-[1-(3-cyclopentyloxy-4-methoxyphenyl)-2-(4-pyridyl)ethenyl]phenyl}carbamate;
N-{4-[1-(3-cyclopentyloxy-4-methoxyphenyl)-2(4-pyridyl)ethenyl]phenyl}N'-ethylurea;
N-{4-[1-(3-cyclopentyloxy-4-methoxyphenyl)]-2-(4-pyridyl)ethenyl}phenylacetamide;
3-[2-(3-cyclopentyloxy-4-methoxyphenyl)-2-phenylethenyl]pyridine;
4-[2-(3-cyclopentyloxy-4-methoxyphenyl)-2-phenylethenyl]pyrimidine;
4-[2-(3-cyclopentyloxy-4-methoxyphenyl)-2-(4-hydroxymethylphenyl)ethenyl]pyridine;
4-[1-(3-cyclopentyloxy-4-methoxyphenyl)-2-(4-pyridyl)ethenyl]benzamide;
ethyl-4-[1-(3-cyclopentyloxy-4-methoxyphenyl)-2-(4-phenylethenyl]benzoate;
N-{4-[1-(3-cyclopentyloxy-4-methoxyphenyl)-2-(4-pyridyl)ethenyl]phenyl}methanesulphonamide;or
each (E) or (Z) isomer thereof; and the salts, solvates, hydrate and N-oxides thereof.

8. A pharmaceutical composition comprising a compound according to claim 1 together with one or more pharmaceutically acceptable carriers, excipients or diluents.

## Patentansprüche

1. Verbindung der Formel (1) 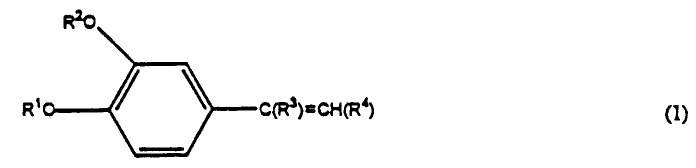
worin R¹ eine Methylgruppe, gegebenenfalls substituiert mit einem, zwei oder drei Fluor- oder Chloratom(en) darstellt;
R² eine C₃₋₈-Cycloalkylgruppe, gegebenenfalls substituiert mit einem, zwei oder drei Substituenten, ausgewählt aus Halogenatomen, einer C₁₋₆-Alkyl-, Hydroxyl- oder C₁₋₆-Alkoxygruppe, darstellt;
R³ und R⁴, die gleich oder verschieden sein können, jeweils eine Gruppe Ar darstellen, worin Ar eine Phenyl-, Pyridyl-, Pyridazinyl-, Pyrimidinyl- oder Pyrazinylgruppe darstellt, wobei jede der Gruppen gegebenenfalls mit einem, zwei oder mehreren Substituenten, ausgewählt aus einem Atom oder einer Gruppe R⁸ oder Alk¹H oder -Alk¹(R⁸)ₘ, worin R⁸ ein Halogenatom, oder eine Amino- (-NH₂), substituierte Amino-, Ni-tro-, Cyano-, Hydroxyl- (-OH), substituierte Hydroxyl-, Cycloalkoxy, Formyl- [HC(O)-] , Carboxyl- (-CO₂H), veresterte Carboxyl-, Thiol- (-SH), substituierte Thiolgruppe oder eine Gruppe -C(O)Alk¹H, -SO₃H, -SO₂Alk¹H, -SO₂NH₂, -SO₂NHAlk¹H, -SO₂N[Alk¹-H]₂, -CONH₂, -CONHAlk¹H, -CON[Alk¹H]₂, -NHSO₂H, -NHSO₂Alk¹H, -N[SO₂Alk¹H]₂ -NHSO₂NH₂, -NHSO₂NHAlk¹H, -NHSO₂N[Alk¹H]₂, -NHC(C)Alk¹H, -NHC(O)OAlk¹H darstellt; Alk¹ eine geradkettige oder verzweigte C₁₋₆-Alkylen-, C₂₋₆-Al-kenylen- oder C₂₋₆-Alkinylenkette, gegebenenfalls unterbrochen mit einem, zwei oder drei Atom(en) -O- oder -S- oder mit den Gruppen -S(O)p-, [worin p die ganze Zahl 1 oder 2 ist] oder N(R⁶)- [worin R⁶ ein Wasserstoffatom oder eine C₁₋₆-Alkylgruppe darstellt] bedeutet;
und m Null oder eine ganze Zahl 1, 2 oder 3 ist;
substituiert sein kann;
wobei die substituierte Aminogruppe eine Gruppe -NH[Alk¹(R^{8a})ₘ], [worin Alk¹ und m wie vorstehend definiert sind und R^{8a} wie vorstehend für R⁸ definiert ist, jedoch keine substituierte Amino-, substituierte Hydroxyl- oder substituierte Thiolgruppe bedeutet] oder eine Gruppe -N[Alk¹(R^{8a})ₘ]₂, worin jede Gruppe -Alk¹(R^{8a})ₘ gleich oder verschieden ist, darstellt;
wobei die substituierte Hydroxyl- oder substituierte Thiolgruppe eine Gruppe -OAlk¹(R^{8a})ₘ bzw. -SAlk¹(R^{8a})ₘ darstellt, worin Alk¹, R^{8a} und m wie vorstehend definiert sind; und die Salze, Solvate, Hydrate und N-Oxide davon.

2. Verbindung nach Anspruch 1, worin R¹ eine Methylgruppe darstellt.

3. Verbindung nach Anspruch 1 oder Anspruch 2, worin R² eine Cyclopencylgruppe darstellt.

4. Verbindung nach einem vorangehenden Anspruch, worin R³ und/oder R⁴ eine substituierte oder unsubstituierte Pyridylgruppe darstellt.

5. Verbindung nach Anspruch 4, worin R³ und R⁴ jeweils eine Pyridyl- oder eine monosubstituierte oder disubstituierte Pyridylgruppe darstellen oder R³ eine Phenyl- oder substituierte Phenylgruppe darstellt und R⁴ eine Pyridyloder eine monosubstituierte oder disubstituierte Pyridylgruppe darstellt.

6. Verbindung nach Anspruch 5, worin die Pyridylgruppen 4-Pyridyl-, 3-monosubstituierte 4-Pyridyl-, 3,5-disubstituierte 4-Pyridyl-, 2- oder 4-monosubstituierte 3-Pyridyloder 2,4-disubstituierte 3-Pyridylgruppen darstellen.

7. Verbindung nach Anspruch 1, ausgewählt aus (E)-und (Z) -Isomeren von:
4-[2-(3-Cyclopentyloxy-4-methoxyphenyl)-2-phenylethenyl]pyridin;
4-[1-(3-Cyclopentyloxy-4-methoxyphenyl)-2-(4-pyridyl)ethenyl]pyridin;
4-[2-(3-Cyclopentyloxy-4-methoxyphenyl)-2-(4-fluorphenylethenyl]pyridin;
4-[2-(3-Cyclopentyloxy-4-methoxyphenyl)-2-(4-trifluormethylphenyl)ethenyl]pyridin;
4-[2-(3-Cyclopentyloxy-4-methoxyphenyl)-2-(2-methoxyphenylethenyl)]pyridin;
4-[2-(3-Cyclopentyloxy-4-methoxyphenyl)-2-(4-methoxyphenyl)ethenyl]pyridin;
4-[2-(3-Cyclopentyloxy-4-methoxyphenyl)-2-(4-methylphenyl)ethenyl]pyridin;
4-[2-(3-Cyclopentyloxy-4-methoxyphenyl)-2-(3-methylphenyl)ethenyl]pyridin;
4-[2-(3-Cyclopentyloxy-4-methoxyphenyl)-2-(3-cyclopentyloxy-4-methoxyphenyl)ethenyl]pyridin;
4-[2-(3-Cyclopentyloxy-4-methoxyphenyl)-2-phenylethenyl]-3,5-dichlorpyridin;
2-[2-(3-Cyclopentyloxy-4-methoxyphenyl)-2-phenylethenyl]pyridin;
4-[1-(3-Cyclopentyloxy-4-methoxyphenyl)-2-(4-pyridyl)ethenyl]anilin;
4-[1-(3-Cyclopentyloxy-4-methoxyphenyl)-2-(4-pyridyl)ethenyl]benzoesäure;
N-{4-[1-(3-Cyclopentyloxy-4-methoxyphenyl)-2-(4-pyridyl)ethenyl]phenyl}carbaminsäureethylester;
N-{4-[1-(3-Cyclopentyloxy-4-methoxyphenyl)-2-(4-pyridyl)ethenyl]phenyl}N'-ethylharnstoff;
N-{4-[1-(3-Cyclopentyloxy-4-methoxyphenyl)]-2-(4-pyridyl)ethenyl}phenylacetamid;
3-[2-(3-Cyclopentyloxy-4-methoxyphenyl)-2-phenylethenyl]pyridin;
4-[2-(3-Cyclopentyloxy-4-methoxyphenyl)-2-phenylethenyl]pyrimidin;
4-[2-(3-Cyclopentyloxy-4-methoxyphenyl)-2-(4-hydroxymethylphenyl)ethenyl]pyridin;
4-[1-(3-Cyclopenzyloxy-4-methoxyphenyl)-2-(4-pyridyl)ethenyl]benzamid;
4-[1-(3-Cyclopentyloxy-4-methoxyphenyl)-2-(4-phenylethenyl]benzoesäureethylester;
N-{4-[1-(3-Cyclopentyloxy-4-methoxyphenyl)-2-(4-pyridyl)ethenyl]phenyl}methansulfonamid oder
jedes (E)- oder (Z)-Isomer davon und die Salze, Solvate, Hydrate und N-Oxide davon.

8. Pharmazeutische Zusammensetzung, umfassend eine Verbindung nach Anspruch 1, zusammen mit einem oder mehreren pharmazeutisch verträglichen Trägern, Exzipienten oder Verdünnungsmitteln.

## Revendications

1. Composé de formule (1) 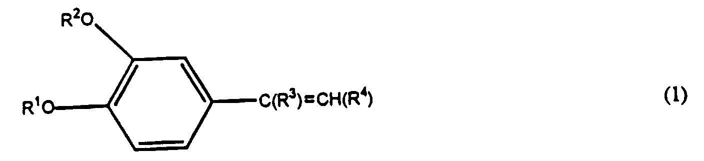
dans laquelle R¹ est un groupe méthyle éventuellement substitué par un, deux ou trois atomes de fluor ou de chlore;
R² est un groupe cycloalkyle en C₃-C₈ éventuellement substitué par un, deux ou trois substituants choisis parmi les atomes d'halogène et un groupe alkyle en C₁-C₆, hydroxyle ou alcoxy en C₁-C₆;
R³ et R⁴, qui peuvent être identiques ou différents, sont chacun un groupe Ar, où Ar est un groupe phényle, pyridyle, pyridazinyle, pyrimidinyle ou pyrazinyle, chacun desdits groupes étant éventuellement substitué par un ou deux substituants, ou plus, choisis parmi un atome ou un groupe R⁸ ou Alk¹H ou -Alk¹(R⁸)ₘ, où R⁸ est un atome d'halogène ou un groupe amino (-NH₂), amino substitué, nitro, cyano, hydroxyle (-OH), hydroxyle substitué, cycloalcoxy, formyle [HC(O)-], carboxyle (-CO₂H), carboxyle estérifié, thiol (-SH), thiol substitué, -C(O)Alk¹H, -SO₃H, -SO₂Alk¹H, -SO₂NH₂, -SO₂NHAlk¹H, -SO₂N[Alk¹H]₂, -CONH₂,-CONHAlk¹H, -CON[Alk¹H]₂, -NHSO₂H, -NHSO₂Alk¹H, -N[SO₂Alk¹H]₂, -NHSO₂NH₂, -NHSO₂NHAlk¹H, -NHSO₂N[Alk¹H]₂, -NHC(O)Alk¹H ou -NHC(O)OAlk¹H; Alk¹ est un groupe alkylène en C₁-C₆, alcénylène en C₂-C₆ ou alcynylène en C₂-C₆ linéaire ou ramifié, éventuellement interrompu par un, deux ou trois atomes -O- ou -S- ou groupes -S(O)ₚ- (où p est un nombre entier égal à 1 ou 2) ou N(R⁶)- (où R⁶ est un atome d'hydrogène ou un groupe alkyle en C₁-C₆);
et m est nul ou un nombre entier égal à 1, 2 ou 3;
ledit groupe amino substitué étant un groupe -NH[Alk¹(R^{8a})ₘ] [où Alk¹ et m sont tels que définis ci-dessus et R^{8a} est tel que défini ci-dessus pour R⁸, mais n'est pas un groupe amino substitué, un groupe hydroxyle substitué ou un groupe thiol substitué] ou un groupe -N[Alk¹(R^{8a})ₘ]₂, où chaque groupe Alk¹(R^{8a})ₘ est identique ou différent; ledit groupe hydroxyle substitué ou thiol substitué étant un groupe -OAlk¹(R^{8a})ₘ ou -SAlk¹(R^{8a})ₘ respectivement, où Alk¹, R^{8a} et m viennent d'être définis; et
leurs sels, solvates, hydrates et N-oxydes.

2. Composé selon la revendication 1, dans lequel R¹ est un groupe méthyle.

3. Composé selon la revendication 1 ou 2, dans lequel R² est un groupe cyclopentyle.

4. Composé selon l'une quelconque des revendications précédentes, dans lequel R³ et/ou R⁴ est un groupe pyridyle substitué ou non substitué.

5. Composé selon la revendication 4, dans lequel R³ et R⁴ sont chacun un groupe pyridyle ou un groupe pyridyle monosubstitué ou disubstitué, ou R³ est un groupe phényle ou phényle substitué et R⁴ est un groupe pyridyle ou un groupe pyridyle monosubstitué ou disubstitué.

6. Composé selon la revendication 5, dans lequel lesdits groupes pyridyle sont les groupes 4-pyridyle, 4-pyridyle 3-monosubstitué, 4-pyridyle 3,5-disubstitué, 3-pyridyle 2- ou 4-monosubstitué ou 3-pyridyle 2,4-disubstitué.

7. Composé selon la revendication 1, choisi parmi les isomères (E) et (Z) de:
la 4-[2-(3 -cyclopentyloxy-4-méthoxyphényl)-2-phényléthényl]pyridine;
la 4-[1-(3 -cyclopentyloxy-4-méthoxyphényl)-2 -(4-pyridyl)éthényl]pyridine;
la 4-[2-(3-cyclopentyloxy-4-méthoxyphényl)-2-(4-fluorophényléthényl]-pyridine;
la 4-[2-(3-cyclopentyloxy-4-méthoxyphényl)-2-(4-trifluorométhylphényl)-éthényl]pyridine;
la 4-[2-(3-cyclopentyloxy-4-méthoxyphényl)-2-(2-méthoxyphényléthényl)]-pyridine;
la 4-[2-(3 -cyclopentyloxy-4-méthoxyphényl)-2-(4-méthoxyphényl)éthényl]-pyridine;
la 4-[2-(3-cyclopentyloxy-4-méthoxyphényl)-2-(4-méthylphényl)éthényl]-pyridine;
la 4-[2-(3-cyclopentyloxy-4-méthoxyphényl)-2-(3-méthylphényl)éthényl]-pyridine;
la 4-[2-(3-cyclopentyloxy-4-méthoxyphényl)-2-(3-cyclopentyloxy-4-méthoxyphényl)éthényl]pyridine;
la 4-[2-(3-cyclopentyloxy-4-méthoxyphényl)-2-phényléthényl]-3,5-dichloropyridine;
la 2-[2-(3-cyclopentyloxy-4-méthoxyphényl)-2-phényléthényl]pyridine;
la 4-[1-(3-cyclopentyloxy-4-méthoxyphényl)-2-(4-pyridyl)éthényl]aniline;
l'acide 4-[1-(3-cyclopentyloxy-4-méthoxyphényl)-2-(4-pyridyl)éthényl]-benzoïque;
le N-{4-[1-(3-cyclopentyloxy-4-méthoxyphényl)-2-(4-pyridyl)éthényl]-phényl}carbamated'éthyle;
la N-{4-[1-(3-cyclopentyloxy-4-méthoxyphényl)-2-(4-pyridyl)éthényl]-phényl} -N'-éthylurée;
le N-{4-[1-(3-cyclopentyloxy-4-méthoxyphényl)-2-(4-pyridyl)éthényl}-phénylacétamide;
la 3-[2-(3-cyclopentyloxy-4-méthoxyphényl)-2-phényléthényl]pyridine;
la 4-[2-(3 -cyclopentyloxy-4-méthoxyphényl)-2-phényléthényl]pyrimidine;
la 4-[2-(3-cyclopentyloxy-4-méthoxyphényl)-2-(4-hydroxyméthylphényl)-éthényl]pyridine;
le 4-[1-(3-cyclopentyloxy-4-méthoxyphényl)-2-(4-pyridyl)éthényl]-benzamide;
le 4-[1-(3-cyclopentyloxy-4-méthoxyphényl)-2-(4-phényléthényl]benzoate d'éthyle;
le N-{4-[1-(3-cyclopentyloxy-4-méthoxyphényl)-2-(4-pyridyl)éthényl]-phényl} méthanesulfonamide; ou
chacun de leurs isomères (E) ou (Z); et leurs sels, solvates, hydrates et N-oxydes.

8. Composition pharmaceutique comprenant un composé selon la revendication 1 ainsi qu'un ou plusieurs véhicules, excipients ou diluants pharmacologiquement acceptables.
